# EUROPEAN PATENT APPLICATION

(11) **EP 4 624 456 A1**
(43) Date of publication of application: **01.10.2025**
(21) Application number: 24167860.6
(22) Date of filing: 29.03.2024
(51) Int. Cl.: C07D 211/16, C07C 17/35, C07C 25/02, C07C 25/06, C07C 25/10, C07C 25/18, C07C 33/22, C07C 47/54

(54) **PROCESS FOR THE DEAMINATIVE HALOGENATION OF AN AROMATIC OR HETEROAROMATIC AMINO COMPOUND**

(71) Applicant: Studiengesellschaft Kohle gGmbH, 45470 Mülheim (DE)
(72) Inventor: LIST, Benjamin, 45470 Mühlheim an der Ruhr (DE); LOPEZ, Javier Mateos, 45468 Mühlheim an der Ruhr (DE); SCHULTE, Tim, 50937 Köln (DE); RITTER, Tobias, 45470 Mühlheim an der Ruhr (DE)

(57) **Abstract**

The present invention refers to a process for the functionalization such as a deaminative halogenation of an aromatic or heteroaromatic amino compound. With the inventive process, the in-situ nitrate reduction allows the direct ipso-halogenation of anilines and aminoheterocycles via an aryldiazonium or heteroaryl diazonium salt as a fleeting intermediate in solution.

## Description

The present invention refers to a process for the functionalization such as a deaminative halogenation of an aromatic or heteroaromatic amino compound. With the inventive process, the in-situ nitrate reduction allows the direct ipso-halogenation of anilines and aminoheterocycles via an aryldiazonium or heteroaryl diazonium salt as a fleeting intermediate in solution.

Aryldiazonium salts have become a staple in organic synthesis since their discovery in 1858; their synthesis still follows the protocol developed in the 19^{th} century. Due to the favorable reactivity that often cannot be achieved with other aryl(pseudo)halides, diazonium chemistry continues to grow. Facile extrusion of dinitrogen contributes to the desired reactivity but is also reason for safety concerns. Explosions have occurred since their discovery and still result in accidents with casualties that deter from the utility of aryldiazonium salts. Here we report a diazonium chemistry paradigm shift based on nitrate reduction that avoids diazonium accumulation. Because nitrate reduction is rate-limiting, aryldiazoniums are produced as fleeting intermediates, which results in a safer and often more efficient deaminative halogenation in a single step from anilines.

In the prior art, several attempts of using diazonium salts have been made. Sandmeyer used aryldiazonium salts 140 years ago to achieve the oxidative addition to *d*¹⁰ metals for the synthesis of aryl halides. The reactivity of diazonium salts often surpasses that of other (pseudo)halides and is based on their high electrophilicity, low reduction potential of - 0.16 V versus (vs) standard calomel electrode (SCE), and formation of dinitrogen as a leaving group that enables challenging transformations such as single electron transfer-based oxidative addition to main group elements. Therefore, diazonium salts are still prepared routinely both on laboratory scale and in industrial plants; and arylhalides are still commonly prepared from aryldiazonium salts.

Since the first report on aryldiazonium salt synthesis with nitrous acid, little has changed in their preparation. Nitrosonium (NO⁺) ions, formed through acidic degradation of nitrite-based reagents such as sodium- (NaNO₂) or isoamylnitrite, react with anilines at low temperature, which is commonly required due to the instability of aryldiazonium salts above 5 °C. The resulting salts are either isolated, or accumulate for subsequent treatment with nucleophiles, sometimes at higher temperatures, in a separate second step. Reaction enthalpies of up to ΔH= - 84 kcal mol⁻¹ are common for diazonium decomposition reactions and can result in violent degradation. The often unpredictable stability of aryldiazonium salts can result in detonation upon contact with air and even in solution, which has led to a multitude of severe accidents in undergraduate lab courses, academic research, and industry, with fatal consequences at times. The persistent assumption in the community that the tetrafluoroborate (BF₄⁻) counterion prevents explosions of diazonium salts has been proven to be incorrect since BF₄⁻ salts have also been reported to explode.

To attenuate the risks associated with aryldiazonium batch processes, continuous flow set ups can be employed. Although several large scale processes are in place, such as the Balz-Schiemann reaction for the synthesis of fluorobenzene, small changes in the protocol, small quantities of impurities, or mechanical force can result in unexpected detonations, and every new aryldiazonium salt should be considered dangerous and explosive until proven otherwise. To mitigate the dangers associated with diazonium chemistry, alternative deamination protocols have been developed that, for example, employ pyridines as leaving groups or isodiazenes as intermediates, yet, those reactions typically do not achieve the broad utility and high atom economy of the parent diazotization methods.

Thus, there is the need to provide a process for functionalization such as halogenation of an aromatic or heteroaromatic amino compound without the danger of using accumulated explosive diazo compounds.

The problem of the present invention is solved by the provision of the inventive process as defined in the claims. In more detail, the present invention refers to a process for the functionalization such as deaminative halogenation of an aromatic or heteroaromatic amino compound wherein an aromatic or heteroaromatic amino compound is reacted with a compound pair, selected from a pair of an (alkali metal or ammonium) nitrate or mixtures thereof and a sulfur oxygen compound as reductant such as an alkali metal thiosulfate or mixtures thereof or a pair of a nitrate ester of an alkyl alcohol and a dihalocuprate which may be prepared in situ, in the presence of a halogen source, selected from halogen salts or halogen alkyl compounds, whereby a halogenated aromatic or heteroaromatic compound is obtained.

In another embodiment slightly modified with respect to the method described before, the present invention is directed to a process for the deaminative halogenation of an aromatic or heteroaromatic amino compound wherein an aromatic or heteroaromatic amino compound is reacted, in an optionally aqueous polar organic solvent, with a nitrate compound, selected from (alkali metal or ammonium) nitrate salts or nitrate esters of alkyl alcohols, in the presence of a reductant and at last one halogen source, selected from halogens, (alkali metal or ammonium) halogen salts or halogen alkyl compounds, whereby a halogenated aromatic or heteroaromatic compound is obtained.

In either embodiment, the process is characterized by an aryldiazonium or heteroaryl diazonium salt compound as a fleeting intermediate in solution.

In one embodiment of the inventive process, an aromatic or heteroaromatic amino compound is reacted with an (alkali metal or ammonium) nitrate or mixtures thereof and a sulfur oxygen compound as reductant such as an alkali metal thiosulfate or mixtures thereof, in the presence of a halogen source, selected from halogen salts or halogen alkyl compounds, whereby a halogenated aromatic or heteroaromatic compound is obtained.

The sulfur oxygen reductant can be selected from alkali metal or ammonium salts of thiosulfates, such as sodium thiosulfate, thiosulfuric acid, polythionates, dithionates, trithionates, tetrathionates, sulfites, bisulfites, sulfur dioxide, or metabisulfites and mixtures thereof.

The halogen source can be any halogen compound which is capable of transferring a halogen to the aromatic or heteroaromatic amino compound for replacing the diazo group in the fleeting intermediate, and can be selected from halogen salts such as alkali halides or halogen alkyl compounds, whereby a halogenated aromatic or heteroaromatic compound is obtained.

In another embodiment of the inventive process, an aromatic or heteroaromatic amino compound is reacted with a nitrate ester of an alkyl alcohol and a dihalocuprate which may be formed in situ, also in the presence of a halogen source, selected from halogen salts or halogen alkyl compounds, whereby a halogenated aromatic or heteroaromatic compound is obtained.

The inventive method is preferably carried out at an elevated temperature, more preferably at the boiling point of the solvent, usually in the range of 40°C to 120°C. The polar organic solvent is not particularly critical and is preferably selected from the group consisting of polar aprotic solvents such as, but not limited to: acetonitrile, butyronitrile, ethylacetate, chloroform, 1,2-dichloroethane, dichloromethane, acetone, or mixtures thereof, optionally in combination with water up to 50 vol.-% of the liquid phase.

For the inventive method, the process can be carried out under an ambient atmosphere or in a closed container. The process can be carried out under a protective atmosphere such as nitrogen, argon or mixtures thereof, but there is no need in view of the reactions partners.

In the context of the aspects of the present invention, the following definitions are more general terms which are used throughout the present application.

When a range of values is listed, it is intended to encompass each value and sub-range within the range. For example "C₁₋₆" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₆, C₃₋₄, C₄₋₆, C₄₋₆, and C₅₋₆.

The term "aliphatic" includes both saturated and unsaturated, straight chain (i.e., unbranched), branched, acyclic, cyclic, or polycyclic aliphatic hydrocarbons, which are optionally substituted with one or more functional groups. As will be appreciated by one of ordinary skill in the art, "aliphatic" is intended herein to include, but is not limited to, alkyl, alkenyl, alkynyl, cycloalkyl, cycloalkenyl, and cycloalkynyl moieties. Thus, the term "alkyl" includes straight, branched and acyclic alkyl groups. An analogous convention applies to other generic terms such as "alkenyl", "alkynyl", and the like. Furthermore, the terms "alkyl", "alkenyl", "alkynyl", and the like encompass both substituted and unsubstituted groups. In certain embodiments, "lower alkyl" is used to indicate those alkyl groups (acyclic, substituted, unsubstituted, branched or unbranched) having 1-6 carbon atoms.

As used herein, "alkyl" refers to a radical of a straight-chain, branched or cyclic saturated hydrocarbon group having from 1 to 20 carbon atoms ("C₁₋₂₀ alkyl"). In some embodiments, an alkyl group has 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). In some embodiments, an alkyl group has 2 to 6 carbon atoms ("C₂₋₆ alkyl"). Examples of C₁₋₆ alkyl groups include methyl (C₁), ethyl (C₂), n-propyl (C₃), isopropyl (C₃), n-butyl (C₄), tert-butyl (C₄), sec-butyl (C₄), iso-butyl (C₄), n-pentyl (C₅), 3-pentanyl (C₅), amyl (C₅), neopentyl (C₅), 3-methyl-2-butanyl (C₅), tertiary amyl (C₅), and n-hexyl (C₆). Additional examples of alkyl groups include n-heptyl (C₇), n-octyl (C₈) and the like. Unless otherwise specified, each instance of an alkyl group is independently unsubstituted (an "unsubstituted alkyl") or substituted (a "substituted alkyl") with one or more substituents. In certain embodiments, the alkyl group is an unsubstituted C₁₋₁₀ alkyl (e.g., -CH₃). In certain embodiments, the alkyl group is a substituted C₁₋₁₀ alkyl.

In the inventive process, any aromatic or heteroaromatic amino compound having 5 to 30 carbon atoms, optionally having side chains with up to 20 carbon atoms, and optionally up to three heteroatoms selected from O, S or N in the ring structure can be used as starting material, said ring structure having at least one amino substituent group on the aromatic or heteroaromatic ring system which amino substituent group can be reacted to a diazo group and subsequently replaced by a halogen. Said halogen can be any of Cl, Br or I and may be simply added, depending on the halogen, to the solution in the form of an alkali or ammonium salt, as halogen, or as an halogen alkyl compound such as 1,2-dichloroethane, 1,2-dibromoethane or 1,2-diiodoethane.

Said aromatic or aryl refers to a radical of a monocyclic or polycyclic (e.g., bicyclic or tricyclic) 4n+2 aromatic ring system (e.g., having 6, 10, or 14 pi electrons shared in a cyclic array) having 6-14 ring carbon atoms and zero heteroatoms provided in the aromatic ring system ("C₆₋₁₄ aryl"). In some embodiments, an aryl group has six ring carbon atoms ("C₆ aryl"; e.g., phenyl). In some embodiments, an aryl group has ten ring carbon atoms ("C₁₀ aryl"; e.g., naphthyl such as 1-naphthyl and 2-naphthyl). In some embodiments, an aryl group has fourteen ring carbon atoms ("C₁₄ aryl"; e.g., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Unless otherwise specified, each instance of an aryl group is independently optionally substituted, i.e., unsubstituted (an "unsubstituted aryl") or substituted (a "substituted aryl") with one or more substituents. In certain embodiments, the aryl group is unsubstituted C₆₋₁₄ aryl. In certain embodiments, the aryl group is substituted C₆₋₁₄ aryl.

"Aralkyl" is a subset of alkyl and aryl and refers to an optionally substituted alkyl group substituted by an optionally substituted aryl group. In certain embodiments, the aralkyl is optionally substituted benzyl. In certain embodiments, the aralkyl is benzyl. In certain embodiments, the aralkyl is optionally substituted phenethyl. In certain embodiments, the aralkyl is phenethyl.

"Heteroaryl" preferably refers to a radical of a 5-14 membered monocyclic or bicyclic 4n+2 aromatic ring system (e.g., having 6 or 10 pi electrons shared in a cyclic array) having ring carbon atoms and 1-4 ring heteroatoms provided in the aromatic ring system, wherein each heteroatom is independently selected from nitrogen, oxygen, and sulfur ("5-14 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (e.g., indolyl, quinolinyl, carbazolyl, and the like) the point of attachment can be on either ring, i.e., either the ring bearing a heteroatom (e.g., 2-indolyl) or the ring that does not contain a heteroatom (e.g., 5-indolyl).

In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur. Unless otherwise specified, each instance of a heteroaryl group is independently optionally substituted, i.e., unsubstituted (an "unsubstituted heteroaryl") or substituted (a "substituted heteroaryl") with one or more substituents. In certain embodiments, the heteroaryl group is unsubstituted 5-14 membered heteroaryl. In certain embodiments, the heteroaryl group is substituted 5-14 membered heteroaryl.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

The present invention is further illustrated by the attached Figures and Examples. In the Figures, it is shown:

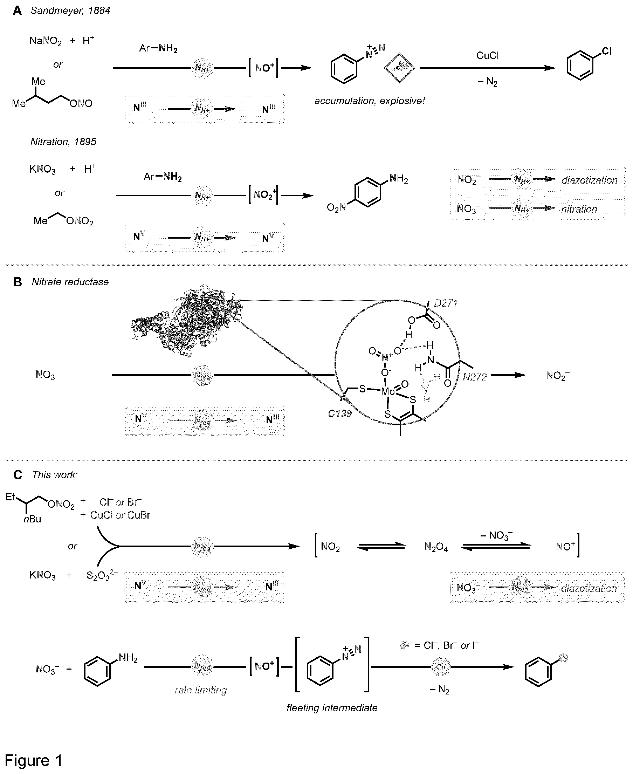

**Fig. 1****. Diazonium salts as fleeting intermediates**
   **(A)** Top: acidic activation of nitrite and alkylnitrites leads to accumulation of aryldiazonium salts; bottom: acidic activation of nitrate and nitrate esters leads to nitration.
   **(B)** Nitrate reductase metalloenzymes use nitrate as terminal electron acceptor for respiration.
   **(C)** This work: nitrate reduction with thiosulfate or cuprates as rate limiting step enables direct functionalization of anilines. Me = methyl, Ar = aryl, N_{H+} = Nitrogen oxyanion protonation, N_{red} = Nitrate reduction.

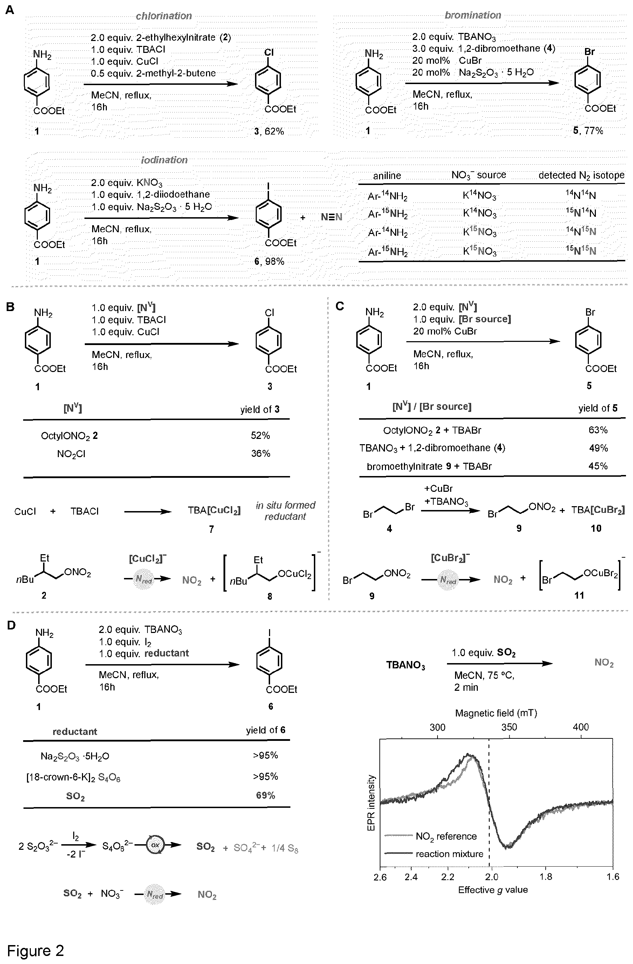

**Fig. 2****. Mechanistic insights for direct deaminative halogenations by nitrate reduction.**
   **(A)** Optimized conditions for chlorination, bromination, and iodination of **1**; nitrogen detection by GCMS and isotopic labeling.
   **(B)** Nitrate ester reduction in deaminative chlorination proceeds by single electron transfer (SET) from in situ formed dichlorocuprate **7.**
   **(C)** Formation of nitrate ester **9** from 1,2-dibromoethane **4** followed by SET from dibromocuprate **10** in deaminative bromination.
   **(D)** For the deaminative iodination reaction, SO₂ is responsible for nitrate reduction, which is formed from thiosulfate; detection of NO₂ radical using EPR spectroscopy, for spectroscopic details and EPR parameters, see Fig. S47; Me = methyl, Et = ethyl, Ph = phenyl, TBA = tetrabutylammonium, MeCN = acetonitrile, 18-crown-6 = 1,4,7,10,13,16-hexaoxacyclooctadecane (18-crown-6 ether), ox = oxidant, N_{red} = Nitrate reduction; n.r. = <5% product detected by ¹H-NMR spectroscopy.

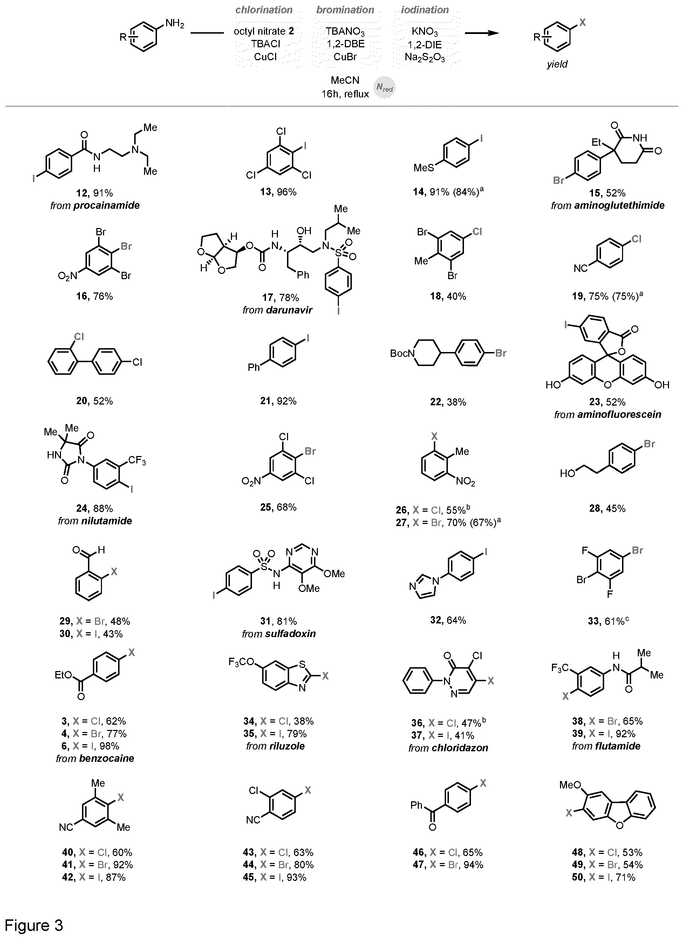

**Fig. 3****. Substrate scope of anilines.** Reaction conditions: chlorination; 0.500 mmol aniline, 1.00 mmol **2**, 0.500 mmol CuCl, 0.500 mmol TBACI, 0.250 mmol 2-methyl-2-butene in 1.25 mL of acetonitrile, reflux, 16h; bromination: 0.500 mmol aniline, 1.00 mmol TBANO₃, 0.100 mmol CuBr, 0.100 mmol Na₂S₂O₃·5H₂O, 1.50 mmol 1,2-dibromoethane in 1.25 mL of acetonitrile, reflux, 16h; iodination: 0.500 mmol aniline, 1.00 mmol KNO₃, 0.600 mmol Na₂S₂O₃·5H₂O, 0.600 mmol 1,2-diiodoethane in 1.25 mL of acetonitrile, reflux, 16h. ^{a.}10.0 mmol scale. ^{b.}2.00 mmol **2** and 1.00 mmol CuCI. ^{c.1}H-NMR yield due to the volatility of compound **33.** See supporting information for detailed experimental procedures. Me = methyl, Et = ethyl, Ph = phenyl, TBA = tetrabutylammonium, N_{red} = Nitrate reduction.

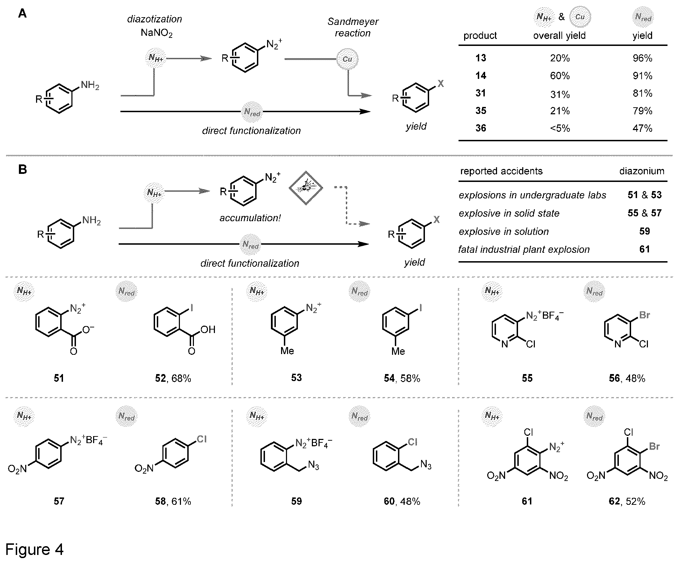

**Fig. 4****. Direct halogenation of anilines of which diazonium salts are reported explosives.**
   **(A)** Comparison of two-step synthesis of aryl halides with the nitrate-based protocol.
   **(B)** Direct halogenation of anilines of which diazonium salts are reported explosives.

Reaction conditions: chlorination; 0.500 mmol aniline, 1.00 mmol **2**, 0.500 mmol CuCl, 0.500 mmol TBACI, 0.250 mmol 2-methyl-2-butene in 1.25 mL of acetonitrile, reflux, 16h; bromination: 0.500 mmol aniline, 1.00 mmol TBANO₃, 0.600 mmol CBr₄ and 0.400 mmol Na₂S₂O₃·5H₂O in 1.25 mL of ethylacetate, reflux, 16h; iodination: 0.500 mmol aniline, 1.00 mmol KNO₃, 0.600 mmol Na₂S₂O₃·5H₂O, 0.600 mmol 1,2-diiodoethane in 1.25 mL of acetonitrile, reflux, 16h. See supporting information for detailed experimental procedures. Me = methyl, TBA = tetrabutylammonium, N_{H+} = Nitrogen oxyanion protonation, N_{red} = Nitrate reduction

The inventors started their work with deliberations concerning a nitrate reduction strategy. According to said considerations, the formation of NO₂⁺ in acidic conditions and the kinetic stability of NO₃⁻ may be the reasons that nitrate salts and their esters have been ignored as diazotization reagents over more than a century. The conversion of NO₂⁻ to NO⁺, and of NO₃⁻ to NO₂⁺ with acid are redox neutral processes. Use of nitrate (N oxidation state = +V) as diazotization reagent therefore requires a reductive pathway in the absence of strong acid for the generation of NO⁺ (N oxidation state = +III). Single electron reduction to nitrogen dioxide (NO₂, N oxidation state = +IV) allows the formation of NO⁺ through dimerization and then disproportionation of dinitrogen tetroxide (N₂O₄). The inventor's protocol relies on thiosulfate or dihalocuprates as electron donors for nitrate reduction and can employ readily available potassium nitrate or nitrate esters. Although NO₃⁻ is thermodynamically strong enough to oxidize nucleophiles like bromide and iodide based on reduction potentials, it is compatible with various nucleophiles in the same reaction mixture, including bromide, iodide, and thiosulfate. In contrast, the hitherto used nitrite and alkylnitrites are incompatible with several nucleophiles because they react by kinetically facile oxidation. Therefore, such nucleophiles cannot be present during diazotization with nitrites; and diazoniums accumulate or must be isolated, which results in the safety issues of global diazonium chemistry.

Because the process of nitrate reduction is the rate-limiting step in the inventor's strategy, diazoniums are produced as fleeting intermediates in only minute concentrations, which results in several fundamental advantages when compared to the conventional diazonium chemistry employed over the past 150 years: i) exothermic decomposition and fast nitrogen evolution of aryldiazoniums is avoided, ii) anilines are used as starting materials directly, which avoids a two-step sequence with isolation or storage of diazonium salts, iii) most aryldiazoniums are unstable above 5 °C, therefore, conventional reactions are generally restricted to low temperature reactions, yet, the inventor's protocol is not limited by such a constraint and may access reaction chemistry beyond traditional diazonium reactions, and iv) nitrate and its esters are more readily available and less expensive than alkylnitrites. In addition, diazotization and functionalization of aryldiazoniums produce H₂O and N₂ as byproducts, as compared to other, modern deaminative strategies with pyridines, carboxylates, or esters as side products.

Thus, the inventors describe the new organic and inorganic chemistry for the direct deaminative halogenation of anilines via aryldiazoniums as fleeting intermediates, using abundant nitrate salts and nitrate esters, readily available reductants, and common halide sources. Chlorination can be achieved using tetrabutylammonium chloride (TBACI), CuCI, and nitrate esters - such as 2-ethylhexyl nitrate 2, which is obtained from 2-ethylhexanol, a common plasticizer alcohol produced on multimillion ton scale every year by the Guerbet reaction. Bromination is performed using TBANO₃, Na₂S₂O₃·5H₂O, 1,2-dibromoethane 4 (DBE) as bromine source, and CuBr. Iodination is carried out using KNO₃, sodium thiosulfate pentahydrate (Na₂S₂O₃·5H₂O) as reductant, and 1,2-diiodoethane (DIE) as iodine source. While all three deaminative halogenations reported here can be carried out using both nitrate salts and esters under the same reaction conditions, chlorination delivered higher yields with 2-ethylhexyl nitrate 2, while bromination and iodination resulted in higher yields with nitrate salts. In conventional diazotization methods, halides are oxidized by nitrites when mixed simultaneously, while the use of nitrates allows the presence of nucleophiles to deliver aryl halides in a single step (Fig. 2A).

As regards the scope and a comparison to conventional diazonium chemistry, a variety of structurally and electronically diverse arenes and hetarenes can participate in the nitrate-reduction-based deaminative halogenation (Fig. 3). Substrate-dependent optimization of the reaction conditions was not required during the preparation of the substrate scope. The reactions were set up with technical grade solvents, commercially available starting materials and reagents, and without the need of flow equipment. An inert atmosphere is not required; the reaction is conducted in a closed system under air, and 50 volume% of water content in the solvent still resulted in 70% yield vs 98% yield with ~5 volume% water content. The nitrogen-based species that are formed during the reaction and that are responsible for the generation of NO⁺ are gaseous, therefore the reactions were preferably carried out in closed systems. However, the use of sealed vessels is not a requirement for deaminative chlorination and bromination. Deaminative iodination requires a closed system, but can proceed at pressures lower than 3.1 bars and variation of the reactor size and headspace volume did not change the reaction yield significantly. The distinct mechanism of diazotization via nitrate reduction can address several limitations of conventional diazonium chemistry that include: undesired oxidation of functional groups; failure to efficiently functionalize sterically hindered anilines and aminoheterocycles; low product yield due to competing biaryl and phenol formation through radical dimerization and addition of water, respectively; and undesired protodeaminations. The disadvantages result from the use of nitrites and the high concentration of the reactive diazonium salt that lead to side reactions, which is not the case when diazoniums are produced as fleeting intermediates, in the absence of strong, kinetically reactive oxidants like nitrite. With nitrate salts and nitrate ester 2, oxidant-labile thioethers are tolerated (14); functionalization of *ortho* and *ortho,ortho-*disubstituted anilines (13) proceeds in up to 96% yield; and aminoheterocycles or anilines with heterocyclic substituents (**31, 35, 36**) can participate (Fig. 4A). Improvement over conventional diazonium chemistry becomes even more apparent in a comparison of severely dangerous, explosive diazonium salts (Fig. 4B). Anthranilic acid and meta-toluidine are substrates for diazonium preparation in undergraduate labs, although both diazonium salts have detonated. Pyridine-based diazoniums are often unstable in the solid state. Aryldiazoniums that are classified as potentially explosive by the rule of six- because they contain more than one energetic functional group per six carbon atoms - have caused explosions when dry and even in solution, as for example nitroarene **57** or azide **59.** For dinitro-substituted diazonium salt **61** a fatal explosion killed three and injured 31 individuals in a Ciba AG plant. The nitrate reduction strategy avoids accumulation of the explosive diazonium salts and gives direct access to the aryl halides without observation of any violent reaction.

### Materials and Methods

All air- and moisture-insensitive reactions were carried out under ambient atmosphere and monitored by thin-layer chromatography (TLC). High-resolution mass spectra were obtained using *Q Exactive Plus* from *Thermo.* Concentration under reduced pressure was performed by rotary evaporation at 25-40°C at an appropriate pressure. Purified compounds were further dried under high vacuum (0.010-0.005 mbar). Yields refer to purified and spectroscopically pure compounds, unless otherwise stated.

### Solvents

Acetonitrile was purchased from Fisher Scientific. Anhydrous solvents were obtained from Phoenix Solvent Drying Systems. All deuterated solvents were purchased from Euriso-Top.

### Chromatography

Thin layer chromatography (TLC) was performed using EMD TLC plates pre-coated with 250 µm thickness silica gel 60 F254 plates and visualized by fluorescence quenching under 254 nm UV light or permanganate stain. Flash chromatography was performed using silica gel (40-63 µm particle size) purchased from Geduran^{®}.

### Electrochemistry

For analytical experiments, a BASI^{™} epsilon E2 device was used in combination with a BASI Cell Stand C3, purchased from Bioanalytic systems Inc. For cyclic voltammetry a MF-2013 electrode (99.95 % Pt, 1.6 mm diameter) from Bioanalytic systems Inc. was used as working electrode. Potentials were measured versus a Ag/AgCl/NaCl(aq) (3 M) reference electrode, MF-2052 electrode RE-5B form Bioanalytic systems Inc.

### EPR Spectroscopy

Room temperature CW EPR measurements were carried out at X-band (-9.45 GHz) on a Bruker Magnettech^{™} ESR5000 benchtop spectrometer equipped with a temperature-control system. A 100 kHz modulation frequency with an amplitude of 0.25 mT was used with a microwave power of ~18 mW. Each scan over the field range (187-487 mT) spectra were recorded for 180 s and were accumulated if needed. A digital RC filter with a time constant of 500 ms, implemented on ESR5000, was applied to the raw data. Both post-processing treatments were performed without artificial signal distortion. The spectra after smoothing with the Savitzky-Golay moving averages and correcting the baseline with the use of *"garlic"* function of Easyspin , MATLAB toolbox was used for further analysis.

### NMR Spectroscopy

NMR spectra were recorded on a Bruker AVANCE III HD 500 spectrometer operating at 500 MHz, 471 MHz, and 126 MHz, for ¹H, ¹⁹F, and ¹³C acquisitions, respectively; a Bruker AVANCE NEO 600 spectrometer equipped with a cryogenically cooled cryoBBO probe operating at 600 MHz and 61 MHz for ¹H and ¹⁵N acquisitions, respectively; or on a Bruker AVANCE III HD 400 spectrometer operating at 400 MHz, 100 MHz, 29 MHz, and 54 MHz for ¹H, ¹³C, ¹⁴N, and ¹⁷O acquisitions, respectively. ¹H and ¹³C Chemical shifts are reported in ppm with the solvent residual peak as the internal standard. For ¹H NMR: CDCl₃, δ 7.26; DMSO-*d₆*, δ 2.50; CD₃CN, δ 1.94; For ¹³C NMR: CDCl₃, δ 77.2; DMSO-*d₆*, δ 39.5; CD₃CN, δ 1.3. Data is reported as follows: s = singlet, d = doublet, t = triplet, q = quartet, p = pentet, m = multiplet, br = broad; coupling constants in Hz; integration. ¹⁴N and ¹⁵N shifts are reported relative to MeNO₂ (δ = 0 ppm). ¹⁷O shifts are reported relative to D₂O (δ = 0 ppm). NMR shifts of heteronuclei were referenced using their respective Ξ value using the *xiref* au program in Bruker Topspin following IUPAC recommendations.

### Computational details

All calculations were performed using a development version of the ORCA program package based on version 5.0. Geometry optimizations, transition-state locations, and enthalpy and free energy computations at standard conditions (298.15 K and 1 atm) were performed using the M06-2X-D3(0) density functional with the ma-def2-TZVP basis set, i.e., at the M06-2X-D3(0)/ma-def2-TZVP level. Electronic energies were then refined with the ma-def2-QZVP basis set. In all computations, the acetonitrile solvent effect was incorporated implicitly using the "Solvation Model based on Density (SMD)" scheme.

### Starting materials

All substrates were used as received from commercial suppliers, or prepared according to published procedures, respectively, unless otherwise stated. Anilines, nitrates, and dihaloethanes were purchased from *Sigma-Aldrich, TCI, Alfa Aesar, BLDPharm, or Chemlmpex.*

### Safety statement

The procedures reported in this work are intended for use only by individuals with proper training in experimental chemistry. All hazardous materials (solid, liquid, or gaseous) should be handled using the standard work procedures described in references such as Chapter 4 of "Prudent Practices in the Laboratory". All chemical waste should be disposed of in accordance with local regulations. For general guidelines for the management of chemical waste, see Chapter 8 of "Prudent Practices in the Laboratory". Reaction set-up, and chemical-specific hazards are highlighted in bold with **"Caution:"** notes in the procedures reported in these supplementary materials. It is important to note that the absence of a caution note does not imply that no significant hazards are associated with the chemicals involved in that procedure.

During the course of this study no explosions or violent decompositions occurred. A summary of the possible risks and hazards is described below:

### Experimental Data

### General procedures for deaminative halogenation of anilines with nitrate

**Caution:** When performing reactions in pressurized systems (such as closed vials, pressure tubes, and autoclaves), a blast shield must be used to minimize personal damage in case of an accident.

### General procedure for deaminative iodination

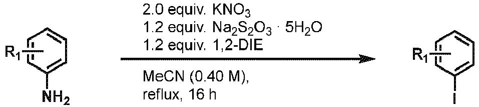

0.5 mmol Scale: Under an ambient atmosphere, to a 4-mL borosilicate vial equipped with a magnetic stir bar were added the (hetero)aromatic amine (if solid, 0.500 mmol, 1.00 equiv.), KNO₃ (101 mg,1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv.), and acetonitrile (1.3 mL, c = 0.40 M).

Then, the (hetero)aromatic amine (if liquid, 0.500 mmol, 1.00 equiv.) was added to the reaction mixture via a Hamilton syringe. The vessel was sealed with a septum cap, and heated at 80°C for 16 h in an aluminum block on a heating plate. Then, the reaction mixture was allowed to cool to 23°C, diluted with dichloromethane (5 mL), and washed with a saturated aqueous Na₂S₂O₃ solution (7 mL). The aqueous phase was extracted with dichloromethane (5 mL). The combined organic phase was dried over Na₂SO₄ and filtered. Then, the resulting filtrate was concentrated by rotary evaporation under reduced pressure. In order to obtain analytically pure samples of aryl iodides, the residue was purified by chromatography on silica gel.

### General procedure for deaminative bromination

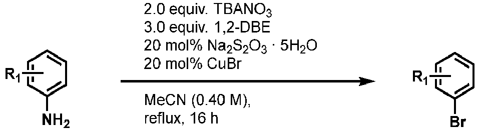

0.5 mmol Scale: Under an ambient atmosphere, to a 4-mL borosilicate vial equipped with a magnetic stir bar were added the (hetero)aromatic amine (if solid, 0.500 mmol, 1.00 equiv.), TBANO₃ (304 mg,1.00 mmol, 2.00 equiv.), CuBr (13 mg, 0.10 mmol, 20 mol%), Na₂S₂O₃·5H₂O (34 mg, 0.10 mmol, 0.20 equiv.), and acetonitrile (1.3 mL, c = 0.40 M). Then, the (hetero)aromatic primary amine (if liquid, 0.500 mmol, 1.00 equiv.), and 1,2-dibromoethane (130 µL, 282 mg, 1.50 mmol, 3.00 equiv.) were added to the reaction mixture via a Hamilton syringe. The vessel was sealed with a septum cap and heated at 80°C for 16 h in an aluminum block on a heating plate. Then, the reaction mixture was allowed to cool to 23°C, diluted with dichloromethane (5 mL), and washed with a saturated aqueous Na₂S₂O₃ solution (7 mL). The aqueous phase was extracted with dichloromethane (5 mL). The combined organic phase was dried over Na₂SO₄ and filtered. Then, the resulting filtrate was concentrated by rotary evaporation under reduced pressure. In order to obtain analytically pure samples of aryl iodides, the residue was purified by chromatography on silica gel.

### General procedure for deaminative chlorination

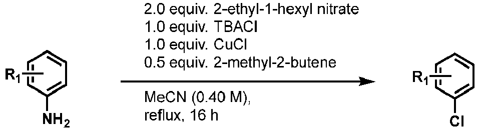

0.500 mmol Scale: Under an ambient atmosphere, to a 4-mL borosilicate vial equipped with a magnetic stir bar were added the (hetero)aromatic amine (if solid, 0.500 mmol, 1.00 equiv.), CuCI (49.5 mg, 0.500 mmol, 1.00 equiv.), TBACI (139 mg, 0.500 mmol, 1.00 equiv.), and acetonitrile (1.3 mL, c = 0.40 M). Then, the (hetero)aromatic primary amine (if liquid, 0.500 mmol, 1.00 equiv.), 2-ethyl-1-hexylnitrate (188µL, 181 mg, 1.00 mmol, 2.00 equiv.), and 2-methyl-2-butene (27 µL, 18 mg, 0.25 mmol, 0.50 equiv.) were added to the reaction mixture via a Hamilton syringe. The vessel was sealed with a septum cap and heated at 80°C for 16 h in an aluminum block on a heating plate. Then, the reaction mixture was allowed to cool to 23°C, diluted with ethyl acetate (5 mL), and washed twice with a saturated aqueous Na₂S₂O₃ solution (2 × 7 mL). The combined organic phase was dried over Na₂SO₄ and filtered. Then, the resulting filtrate was concentrated by rotary evaporation under reduced pressure. In order to obtain analytically pure samples of aryl iodides, the residue was purified by chromatography on silica gel.

### Deaminative halogenations with nitrate salts and nitrate ester

### Ethyl 4-chlorobenzoate (3)

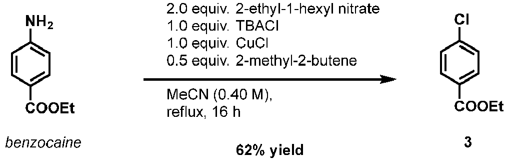

Under an ambient atmosphere, to a 4-mL borosilicate vial equipped with a magnetic stir bar was added benzocaine (82.6 mg, 0.500 mmol, 1.00 equiv), CuCI (49.5 mg, 0.500 mmol, 1.00 equiv.), TBACI (139 mg, 0.500 mmol, 1.00 equiv.), and acetonitrile (1.3 mL, c = 0.40 M). Then, 2-ethyl-1-hexylnitrate (188 µL, 181 mg, 1.00 mmol, 2.00 equiv.), and 2-methyl-2-butene (27 µL, 18 mg, 0.25 mmol, 0.50 equiv.) were added to the reaction mixture via a Hamilton syringe. The vessel was sealed with a septum cap and heated at 80°C for 16 h in an aluminum block on a heating plate. Then, the reaction mixture was allowed to cool to 23°C, diluted with ethyl acetate (5 mL), and washed twice with a saturated aqueous Na₂S₂O₃ solution (2 × 7 mL). The combined organic phase was dried over Na₂SO₄ and filtered. Then, the resulting filtrate was concentrated by rotary evaporation under reduced pressure. The residue was purified by silica gel chromatography by eluting with pentane to afford 57 mg (62%) of the title compound 3 as a colorless oil.

HRMS-ESI(m/z) calc'd for NaC₉H₉O₂Cl⁺ [M+Na]⁺, 207.0183; found, 207.0184; deviation: +0.2 ppm.

### Ethyl 4-bromobenzoate (5)

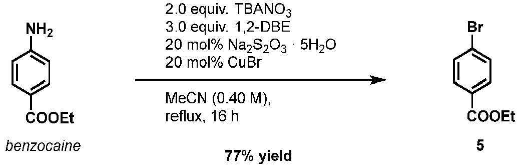

Under an ambient atmosphere, to a 4-mL borosilicate vial equipped with a magnetic stir bar was added benzocaine (82.6 mg, 0.500 mmol, 1.00 equiv), TBANO₃ (304 mg, 1.00 mmol, 2.00 equiv.), CuBr (13 mg, 0.10 mmol, 20 mol%), Na₂S₂O₃·5H₂O (34 mg, 0.10 mmol, 0.20 equiv.), and acetonitrile (1.3 mL, c = 0.40 M). Then, 1,2-dibromoethane (130 µL, 282 mg, 1.50 mmol, 3.00 equiv.) was added to the reaction mixture via a Hamilton syringe. The vessel was sealed with a septum cap and heated at 80°C for 16 h in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with EtOAc/pentane (1:99 (v/v)) to afford 88 mg (77%) of the title compound 5 as a colorless oil. HRMS-ESI(m/z) calc'd for NaC₉H₉O₂Br⁺ [M+Na]⁺, 250.9678; found, 250.9678; deviation: - 0.2 ppm.

### Ethyl 4-iodobenzoate (6)

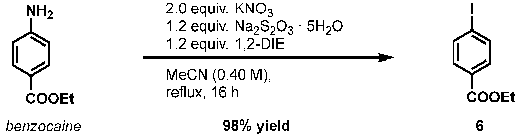

Under an ambient atmosphere, to a 4-mL borosilicate vial equipped with a magnetic stir bar was added benzocaine (82.6 mg, 0.500 mmol, 1.00 equiv.), KNO₃ (101 mg, 1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 ml, c = 0.40 M). The vessel was sealed with a septum cap and heated at 80°C for 16 h in an aluminum block on a heating plate. Then, the reaction mixture was diluted with ethyl acetate (5 ml) and washed with a saturated aqueous Na₂S₂O₃ solution (7 ml). The aqueous phase was extracted with ethyl acetate (5 ml). The combined organic phase was dried over Na₂SO₄ and filtered. The resulting mixture was concentrated by rotary evaporation under reduced pressure. The residue was purified by silica gel chromatography by eluting with EtOAc/pentane (1:99 (v/v)) to afford 134 mg (98%) of the title compound 6 as a colorless oil.

R*f* = 0.55 (EtOAc/pentane (5:95 (v/v)) (UV)).

HRMS-ESI(m/z) calc'd for NaC₉H₉O₂I⁺ [M+Na]⁺, 298.9540; found, 298.9541; deviation: +0.6 ppm.

### Procainamide-derived aryl iodide 12

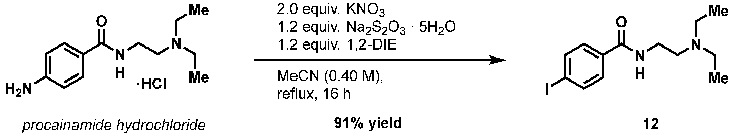

Under an ambient atmosphere, to a 4-mL borosilicate vial equipped with a magnetic stir bar was added procainamide hydrochloride (136 mg, 0.500 mmol, 1.00 equiv.), KNO₃ (101 mg, 1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 mL, c = 0.40 M). The vessel was sealed with a septum cap and heated at 80°C for 16 h in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with pentane to afford 175 mg (91%) of the title compound 12 as light yellow liquid.

Rf= 0.16 (MeOH/CH₂Cl₂ (10:90 (v/v)) (UV)).

HRMS-ESI(m/z) calc'd for C₁₃H₂₀IN₂O⁺ [M+H]⁺, 347.0615; found, 347.0614; deviation: -0.2 ppm.

### 1,3,5-Trichloro-2-iodobenzene (13)

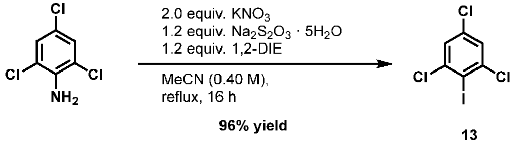

Under an ambient atmosphere, to a 4-mL borosilicate vial equipped with a magnetic stir bar was added 2,4,6-trichloroaniline (98.2 mg, 0.500 mmol, 1.00 equiv.), KNO₃ (101 mg, 1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 mL, c = 0.40 M). The vessel was sealed with a septum cap and heated at 80°C for 16 h in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with pentane to afford 147 mg (96%) of the title compound 13 as a colorless solid.

Rf= 0.80 (in pentane (UV)).

HRMS-EI(m/z) calc'd for C₆H₂Cl₃I⁺ [M]⁺, 305.8261; found, 305.8263; deviation: +0.6 ppm.

### (4-lodophenyl)(methyl)sulfane (14)

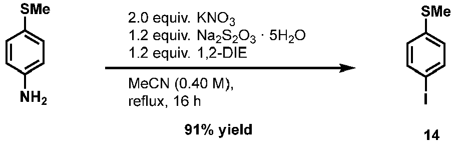

0.5 mmol Scale: Under an ambient atmosphere, to a 4-mL borosilicate vial equipped with a magnetic stir bar was added KNO₃ (101 mg, 1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 mL, c = 0.40 M). Then, 4-(methylthio)aniline (62 □L, 0.50 mmol, 1.0 equiv.) was added to the reaction mixture via a Hamilton syringe. The vessel was sealed with a septum cap and heated at 80°C for 16 h in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with EtOAc/pentane (1:99 (v/v)) to afford 114 mg (91%) of the title compound 14 as a pale orange solid.

### Aminoglutethimide-derived aryl bromide 15

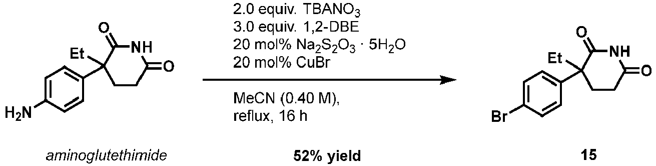

Under an ambient atmosphere, to a 4-mL borosilicate vial equipped with a magnetic stir bar was added aminoglutethimide (116 mg, 0.500 mmol, 1.00 equiv), TBANO₃ (304 mg, 1.00 mmol, 2.00 equiv.), CuBr (13 mg, 0.10 mmol, 20 mol%), Na₂S₂O₃·5H₂O (34 mg, 0.10 mmol, 0.20 equiv.), and acetonitrile (1.3 mL, c = 0.40 M). Then, 1,2-dibromoethane (130 µL, 282 mg, 1.50 mmol, 3.00 equiv.) was added to the reaction mixture via a Hamilton syringe. The vessel was sealed with a septum cap and heated at 80°C for 16 h in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with EtOAc/pentane (20:80 (v/v)) to afford 77 mg (52%) of the title compound 15 as a colorless solid.

R*f* = 0.59 (EtOAc/pentane (40:60 (v/v)) (UV)).

HRMS-ESI(m/z) calc'd for C₁₃H₁₃NO₂Br⁻ [M-H]-, 294.0124; found, 294.0136; deviation: +3.9 ppm.

### 1,2,3-Tribromo-5-nitrobenzene (16)

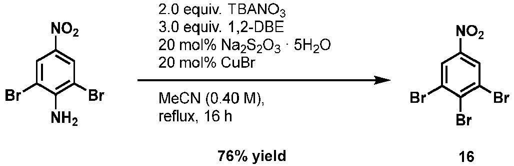

Under an ambient atmosphere, to a 4-mL borosilicate vial equipped with a magnetic stir bar was added 2,6-dibromo-4-nitroaniline (148 mg, 0.500 mmol, 1.00 equiv), TBANO₃ (304 mg,1.00 mmol, 2.00 equiv.), CuBr (13 mg, 0.10 mmol, 20 mol%), Na₂S₂O₃·5H₂O (34 mg, 0.10 mmol, 0.20 equiv.), and acetonitrile (1.3 mL, c = 0.40 M). Then, 1,2-dibromoethane (130 µL, 282 mg, 1.50 mmol, 3.00 equiv.) was added to the reaction mixture via a Hamilton syringe. The vessel was sealed with a septum cap and heated at 80°C for 16 h in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with EtOAc/pentane (2:98 (v/v)) to afford 136 mg (76%) of the title compound 16 as a colorless solid.

Rf= 0.15 (in pentane (UV)).

HRMS-EI(m/z) calc'd for C₆H₂NO₂Br₃⁺ [M]⁺, 356.7631; found, 356.7636; deviation: +1.4 ppm.

### Darunavir-derived aryl iodide 17

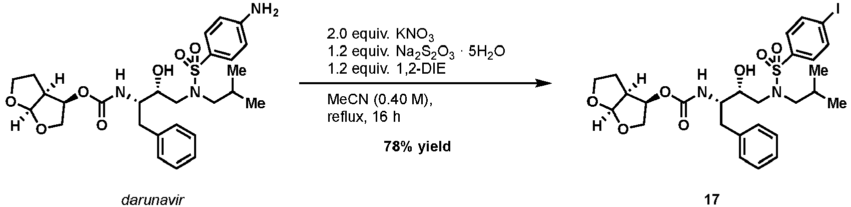

Under an ambient atmosphere, to a 4-mL borosilicate vial equipped with a magnetic stir bar was added darunavir (274 mg, 0.500 mmol, 1.00 equiv.), KNO₃ (101 mg, 1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 mL, c = 0.40 M). The vessel was sealed with a septum cap and heated at 80°C for 16 h in an aluminum block on a heating plate. Then, the reaction mixture was diluted with ethyl acetate (5 mL) and washed with a saturated aqueous Na₂S₂O₃ solution (7 mL). The aqueous phase was extracted with ethyl acetate (5 mL). The combined organic phase was dried over Na₂SO₄ and filtered. The resulting mixture was concentrated by rotary evaporation under reduced pressure. The residue was purified by silica gel chromatography by eluting with EtOAc/pentane (4:6 (v/v)) to afford 257 mg (78%) of the title compound 17 as a colorless solid.

Rf= 0.14 (EtOAc/pentane (40:60 (v/v)) (UV)).

HRMS-ESI(m/z) calc'd for NaC₂₇H₃₅O₇N₂SI⁺ [M+Na]⁺, 681.1102; found, 681.1109; deviation: +1.0 ppm.

### 1,3-Dibromo-5-chloro-2-methylbenzene (18)

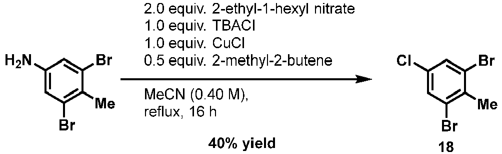

Under an ambient atmosphere, to a 4-mL borosilicate vial equipped with a magnetic stir bar was added 3,5-dibromo-4-methylaniline (131 mg, 0.500 mmol, 1.00 equiv.), CuCI (49.5 mg, 0.500 mmol, 1.00 equiv.), TBACI (139 mg, 0.500 mmol, 1.00 equiv.), and acetonitrile (1.3 mL, *c* = 0.40 M). Then, 2-ethyl-1-hexylnitrate (188 µL, 181 mg, 1.00 mmol, 2.00 equiv.), and 2-methyl-2-butene (27 µL, 18 mg, 0.25 mmol, 0.50 equiv.) were added to the reaction mixture via a Hamilton syringe. The vessel was sealed with a septum cap and heated at 80°C for 16 h in an aluminum block on a heating plate. Then, the reaction mixture was allowed to cool to 23°C, diluted with ethyl acetate (5 mL), and washed twice with a saturated aqueous Na₂S₂O₃ solution (2 × 7 mL). The combined organic phase was dried over Na₂SO₄ and filtered. Then, the resulting filtrate was concentrated by rotary evaporation under reduced pressure. The residue was purified by silica gel chromatography by eluting with pentane to afford 46 mg (40%) of the title compound 18 as a colorless oil.

R*f* = 0.21 (EtOAc/pentane (2:98 (v/v)) (UV)).

HRMS-EI(m/z) calc'd for C₇H₅ClBr₂⁺ [M]⁺, 281.8441; found, 281.8444; deviation: +0.8 ppm.

### 4-Chlorobenzonitrile (19)

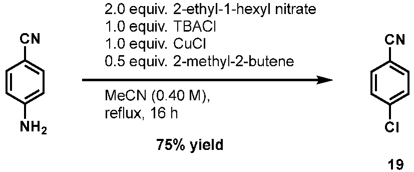

0.5 mmol Scale: Under an ambient atmosphere, to a 4-mL borosilicate vial equipped with a magnetic stir bar was added 4-aminobenzonitrile (59.1 mg, 0.500 mmol, 1.00 equiv), CuCI (49.5 mg, 0.500 mmol, 1.00 equiv.), TBACI (139 mg, 0.500 mmol, 1.00 equiv.), and acetonitrile (1.3 mL, c = 0.40 M). Then, 2-ethyl-1-hexylnitrate (188 µL, 181 mg, 1.00 mmol, 2.00 equiv.), and 2-methyl-2-butene (27 µL, 18 mg, 0.25 mmol, 0.50 equiv.) were added to the reaction mixture via a Hamilton syringe. The vessel was sealed with a septum cap and heated at 80°C for 16 h in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with EtOAc/pentane (5:95 (v/v)) to afford 44 mg (75%) of the title compound 19 as a colorless solid.

### 2,4'-Dichloro-1,1'-biphenyl (20)

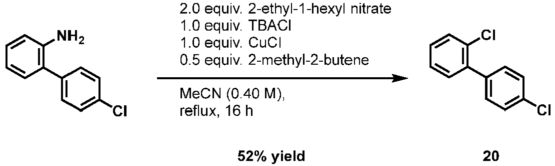

Under an ambient atmosphere, to a 4-mL borosilicate vial equipped with a magnetic stir bar was added 2-amino-4'-chlorobiphenyl (102 mg, 0.500 mmol, 1.00 equiv), CuCI (49.5 mg, 0.500 mmol, 1.00 equiv.), TBACI (139 mg, 0.500 mmol, 1.00 equiv.), and acetonitrile (1.3 mL, *c* = 0.40 M). Then, 2-ethyl-1-hexylnitrate (188 µL, 181 mg, 1.00 mmol, 2.00 equiv.), and 2-methyl-2-butene (27 µL, 18 mg, 0.25 mmol, 0.50 equiv.) were added to the reaction mixture via a Hamilton syringe. The vessel was sealed with a septum cap and heated at 80°C for 16 h in an aluminum block on a heating plate. Then, the reaction mixture was allowed to cool to 23°C, diluted with ethyl acetate (5 mL), and washed twice with a saturated aqueous Na₂S₂O₃ solution (2 × 7 mL). The combined organic phase was dried over Na₂SO₄ and filtered. Then, the resulting filtrate was concentrated by rotary evaporation under reduced pressure. The residue was purified by silica gel chromatography by eluting with pentane to obtain 58 mg (52%) of the title compound 20 as a colorless solid.

R*f* = 0.67 (in pentane (UV))

HRMS-EI(m/z) calc'd for C₁₂H₈Cl₂⁺ [M]⁺, 221.9998; found, 222.0001; deviation: +1.4 ppm.

### 4-lodo-1,1'-biphenyl (21)

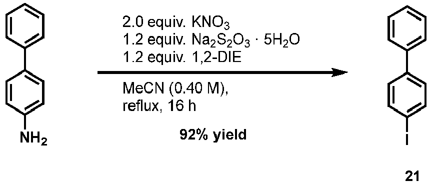

Under an ambient atmosphere, to a 4-mL borosilicate vial equipped with a magnetic stir bar was added 4-aminobiphenyl (84.6 mg, 0.500 mmol, 1.00 equiv.), KNO₃ (101 mg, 1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 mL, c = 0.40 M). The vessel was sealed with a septum cap and heated at 80°C for 16 h in an aluminum block on a heating plate. Then, the reaction mixture was diluted with ethyl acetate (5 mL) and washed with a saturated aqueous Na₂S₂O₃ solution (7 mL). The aqueous phase was extracted with ethyl acetate (5 mL). The combined organic phase was dried over Na₂SO₄ and filtered. The resulting mixture was concentrated by rotary evaporation under reduced pressure. The residue was purified by silica gel chromatography by eluting with EtOAc/pentane (2:98 (v/v)) to afford 127 mg (92%) of the title compound 21 as a colorless solid.

R*f* = 0.46 (EtOAc/pentane (10:90 (v/v)) (UV)).

HRMS-EI(m/z) calc'd for C₁₂H₉I⁺ [M]⁺, 279.9744; found, 279.9742; deviation: -0.4 ppm.

### Tert-butyl 4-(4-bromophenyl)piperidine-1-carboxylate (22)

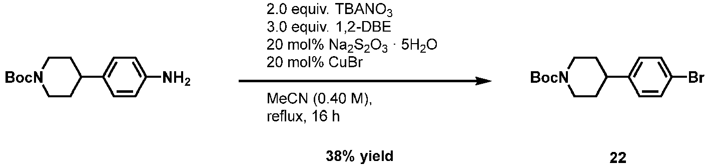

Under an ambient atmosphere, to a 4-mL borosilicate vial equipped with a magnetic stir bar was added tert-butyl 4-(4-aminophenyl)piperidine-1-carboxylate (138 mg, 0.500 mmol, 1.00 equiv.), TBANO₃ (304 mg, 1.00 mmol, 2.00 equiv.), CuBr (13 mg, 0.10 mmol, 20 mol%), Na₂S₂O₃·5H₂O (34 mg, 0.10 mmol, 0.20 equiv.), and acetonitrile (1.3 mL, c = 0.40 M). Then, 1,2-dibromoethane (130 µL, 282 mg, 1.50 mmol, 3.00 equiv.) was added to the reaction mixture via a Hamilton syringe. The vessel was sealed with a septum cap and heated at 80°C for 16 h in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with EtOAc/pentane (5:95 (v/v)) to afford 64 mg (38%) of the title compound 22 as a colorless oil.

R*f* = 0.68 (EtOAc/pentane (20:80 (v/v)) (UV)).

HRMS-ESI(m/z) calc'd for NaC₁₆H₂₂O₂NBr⁺ [M+Na]⁺, 362.0726; found, 362.0728; deviation: +0.5 ppm.

### 5-lodofluorescein (23)

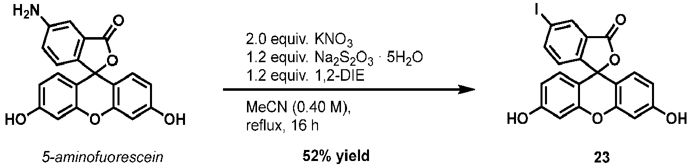

Under an ambient atmosphere, to a 4-mL borosilicate vial equipped with a magnetic stir bar was added 5-aminofluorescein (174 mg, 0.500 mmol, 1.00 equiv.), KNO₃ (101 mg, 1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 mL, c = 0.40 M). The vessel was sealed with a septum cap and heated at 80°C for 16 h in an aluminum block on a heating plate. Then, the reaction mixture was diluted with ethyl acetate (5 mL) and washed with a saturated aqueous Na₂S₂O₃ solution (7 mL). The aqueous phase was extracted with ethyl acetate (5 mL). The combined organic phase was dried over Na₂SO₄ and filtered. The resulting mixture was concentrated by rotary evaporation under reduced pressure. The residue was purified by silica gel chromatography by eluting with EtOAc/pentane (1:1 (v/v)) to afford 120 mg (52%) of the title compound 23 as an orange solid.

R*f* = 0.67 (EtOAc/pentane (70:30 (v/v)) (UV)).

HRMS-ESI(m/z) calc'd for C₂₀H₁₀O₅I⁻ [M-H]-, 456.9579; found, 456.9580; deviation: +0.3 ppm.

### Reduction of nilutamide (S1)

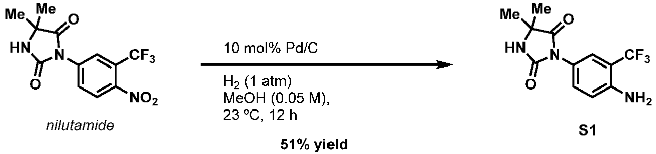

Under an Ar atmosphere, to a 250-mL round-bottomed Schlenk flask equipped with a magnetic stir bar was added nilutamide (600 mg, 1.89 mmol, 1.00 equiv.), and methanol (38 mL, c = 0.05 M). Then, the reaction mixture was stirred at 23°C for 5 min. Pd/C (200 mg, 0.189 mmol, 0.100 equiv.) was added under Ar atmosphere, and the flask was sealed with a septum. The atmosphere was evacuated and a balloon of H₂ was pierced through the septum to ensure a H₂ atmosphere. The reaction mixture was stirred on a hydrogen atmosphere. The reaction was monitored by TLC. After full consumption of the starting material (12 hours), the reaction mixture was filtered through celite. The resulting mixture was concentrated by rotary evaporation under reduced pressure. The residue was purified by silica gel chromatography by eluting with EtOAc/pentane (50:50 (v/v)) to afford 280 mg (51 %) of the title compound S1 as a pale yellow solid.

R*f* = 0.38 (EtOAc/pentane (60:40 (v/v)) (UV)).

HRMS-ESI(m/z) calc'd for NaC₁₂H₁₂N₃O₂F₃⁺ [M+Na]⁺, 310.0774; found, 310.0777; deviation: +0.9 ppm.

### Nilutamide-derived aryl iodide 24

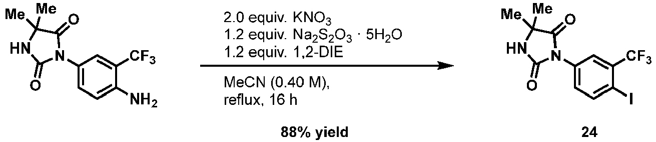

Under an ambient atmosphere, to a 4-mL borosilicate vial equipped with a magnetic stir bar was added S1 (144 mg, 0.500 mmol, 1.00 equiv), KNO₃ (101 mg, 1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 mL, c = 0.40 M). The vessel was sealed with a septum cap and heated at 80°C for 16 h in an aluminum block on a heating plate. Then, the reaction mixture was diluted with ethyl acetate (5 mL) and washed with a saturated aqueous Na₂S₂O₃ solution (7 mL). The aqueous phase was extracted with ethyl acetate (5 mL). The combined organic phase was dried over Na₂SO₄ and filtered. The resulting mixture was concentrated by rotary evaporation under reduced pressure. The residue was purified by silica gel chromatography by eluting with EtOAc/pentane (40:60 (v/v)) to afford 177 mg (88%) of the title compound 24 as a colorless solid.

Rf= 0.45 (EtOAc/pentane (50:50 (v/v)) (UV)).

HRMS-ESI(m/z) calc'd for NaC₁₂H₁₀N₂O₂F₃I⁺ [M+Na]⁺, 420.9631; found, 420.9631; deviation: +0.1 ppm.

### 2-Bromo-1,3-dichloro-5-nitrobenzene (25)

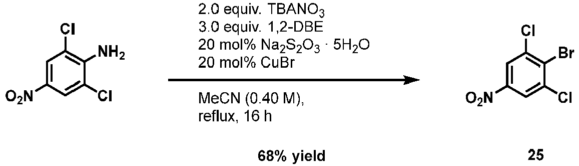

Under an ambient atmosphere, to a 4-mL borosilicate vial equipped with a magnetic stir bar was added 2,6-dichloro-4-nitroaniline (104 mg, 0.500 mmol, 1.00 equiv), TBANO₃ (304 mg, 1.00 mmol, 2.00 equiv.), CuBr (13 mg, 0.10 mmol, 20 mol%), Na₂S₂O₃·5H₂O (34 mg, 0.10 mmol, 0.20 equiv.), and acetonitrile (1.3 mL, c = 0.40 M). Then, 1,2-dibromoethane (130 µL, 282 mg, 1.50 mmol, 3.00 equiv.) was added to the reaction mixture via a Hamilton syringe. The vessel was sealed with a septum cap and heated at 80°C for 16 h in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with EtOAc/pentane (1:99 (v/v)) to afford 93 mg (68%) of the title compound 25 as a colorless solid.

Rf= 0.19 (in pentane (UV)).

HRMS-EI(m/z) calc'd for C₆H₂NO₂BrCl₂⁺ [M]⁺, 268.8641; found, 268.8645; deviation: +1.5 ppm.

### 1-Chloro-2-methyl-3-nitrobenzene (26)

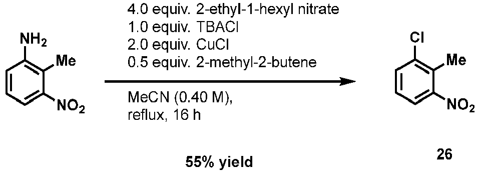

Under an ambient atmosphere, to a 4-mL borosilicate vial equipped with a magnetic stir bar was added 2-methyl-3-nitroaniline (76.1 mg, 0.500 mmol, 1.00 equiv.), CuCI (99.0 mg, 1.00 mmol, 2.00 equiv.), TBACI (139 mg, 0.500 mmol, 1.00 equiv.), and acetonitrile (1.3 mL, c = 0.40 M). Then, 2-ethyl-1-hexylnitrate (375 µL, 361 mg, 2.00 mmol, 4.00 equiv.), and 2-methyl-2-butene (27 µL, 18 mg, 0.25 mmol, 0.50 equiv.) were added to the reaction mixture via a Hamilton syringe. The vessel was sealed with a septum cap and heated at 80°C for 16 h in an aluminum block on a heating plate. Then, the reaction mixture was allowed to cool to 23°C, diluted with ethyl acetate (5 mL), and washed twice with a saturated aqueous Na₂S₂O₃ solution (2 × 7 mL). The combined organic phase was dried over Na₂SO₄ and filtered. Then, the resulting filtrate was concentrated by rotary evaporation under reduced pressure. The residue was purified by silica gel chromatography by eluting with EtOAc/pentane (1:99 (v/v)) to afford 47 mg (55%) of the title compound 26 as a pale yellow oil.

R*f* = 0.65 (EtOAc/pentane (5:95 (v/v)) (UV)).

HRMS-EI(m/z) calc'd for C₇H₆NO₂Cl⁺ [M]⁺, 171.0082; found, 171.0082; deviation: +0.4 ppm.

### 1-Bromo-2-methyl-3-nitrobenzene (27)

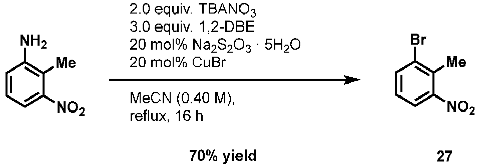

0.5 mmol Scale: Under an ambient atmosphere, to a 4-mL borosilicate vial equipped with a magnetic stir bar was added 2-methyl-3-nitroaniline (76.1 mg, 0.500 mmol, 1.00 equiv.), TBANO₃ (304 mg, 1.00 mmol, 2.00 equiv.), CuBr (13 mg, 0.10 mmol, 20 mol%), Na₂S₂O₃·5H₂O (34 mg, 0.10 mmol, 0.20 equiv.), and acetonitrile (1.3 mL, c = 0.40 M). Then, 1,2-dibromoethane (130 µL, 282 mg, 1.50 mmol, 3.00 equiv.) was added to the reaction mixture via a Hamilton syringe. The vessel was sealed with a septum cap and heated at 80°C for 16 h in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with EtOAc/pentane (1:99 (v/v)) to afford 76 mg (70%) of the title compound 27 as a pale yellow oil.

### 2-(4-Bromophenyl)ethan-1-ol (28)

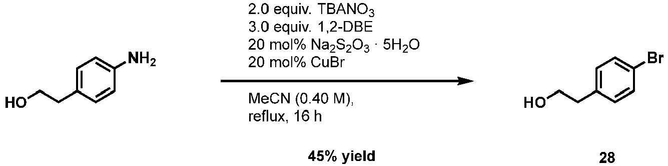

Under an ambient atmosphere, to a 4-mL borosilicate vial equipped with a magnetic stir bar was added 2-(4-aminophenyl)ethan-1-ol (68.5 mg, 0.500 mmol, 1.00 equiv.), TBANO₃ (304 mg, 1.00 mmol, 2.00 equiv.), CuBr (13 mg, 0.10 mmol, 20 mol%), Na₂S₂O₃·5H₂O (34 mg, 0.10 mmol, 0.20 equiv.), and acetonitrile (1.3 mL, c = 0.40 M). Then, 1,2-dibromoethane (130 µL, 282 mg, 1.50 mmol, 3.00 equiv.) was added to the reaction mixture via a Hamilton syringe. The vessel was sealed with a septum cap and heated at 80°C for 16 h in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with EtOAc/pentane (30:70 (v/v)) to afford 45 mg (45%) of the title compound 28 as a yellow oil.

R*f* = 0.24 (EtOAc/pentane (20:80 (v/v)) (UV)).

HRMS-EI(m/z) calc'd for C₈H₉OBr⁺ [M]⁺, 199.9831; found, 199.9834; deviation: +1.0 ppm.

### 2-Bromobenzaldehyde (29)

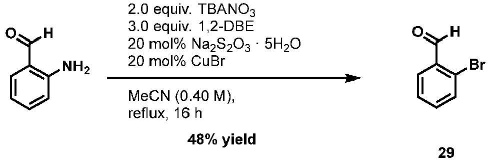

Under an ambient atmosphere, to a 4-mL borosilicate vial equipped with a magnetic stir bar was added 2-aminobenzaldehyde (60.6 mg, 0.500 mmol, 1.00 equiv.), TBANO₃ (304 mg, 1.00 mmol, 2.00 equiv.), CuBr (13 mg, 0.10 mmol, 20 mol%), Na₂S₂O₃·5H₂O (34 mg, 0.10 mmol, 0.20 equiv.), and acetonitrile (1.3 mL, c = 0.40 M). Then, 1,2-dibromoethane (130 µL, 282 mg, 1.50 mmol, 3.00 equiv.) was added to the reaction mixture via a Hamilton syringe. The vessel was sealed with a septum cap and heated at 80°C for 16 h in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with EtOAc/pentane (2:98 (v/v)) to afford 44 mg (48%) of the title compound 29 as a colorless oil.

Rf= 0.55 (EtOAc/pentane (5:95 (v/v)) (UV)).

HRMS-EI(m/z) calc'd for C₇H₅OBr⁺ [M]⁺, 183.9519; found, 183.9521; deviation: +2.0 ppm.

### 2-lodobenzaldehyde (30)

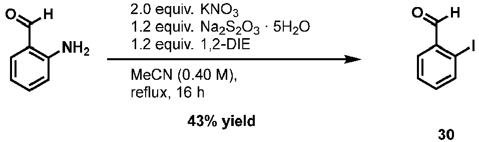

Under an ambient atmosphere, to a 4-mL borosilicate vial equipped with a magnetic stir bar was added 2-aminobenzaldehyde (60.6 mg, 0.500 mmol, 1.00 equiv.), KNO₃ (101 mg, 1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 mL, c = 0.40 M). The vessel was sealed with a septum cap and heated at 80°C for 16 h in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with EtOAc/pentane (3:97 (v/v)) to afford 50 mg (43%) of the title compound 30 as a pale yellow solid.

R*f* = 0.52 (EtOAc/pentane (5:95 (v/v)) (UV)).

HRMS-EI(m/z) calc'd for C₇H₅IO⁺ [M]⁺, 231.9380; found, 231.9384; deviation: +2.0 ppm.

### Sulfadoxin-derived aryl iodide 31

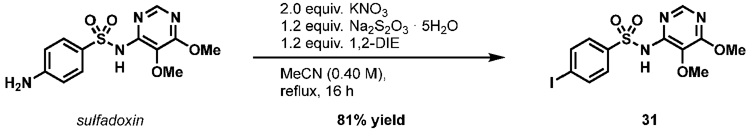

Under an ambient atmosphere, to a 4-mL borosilicate vial equipped with a magnetic stir bar was added sulfadoxin (155 mg, 0.500 mmol, 1.00 equiv.), KNO₃ (101 mg, 1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 mL, c = 0.40 M). The vessel was sealed with a septum cap and heated at 80°C for 16 h in an aluminum block on a heating plate. Then, the reaction mixture was diluted with ethyl acetate (5 mL) and washed with a saturated aqueous Na₂S₂O₃ solution (7 mL). The aqueous phase was extracted with ethyl acetate (5 mL). The combined organic phase was dried over Na₂SO₄ and filtered. The resulting mixture was concentrated by rotary evaporation under reduced pressure. The residue was purified by silica gel chromatography by eluting with EtOAc/pentane (20:80 (v/v)) to afford 170 mg (81%) of the title compound 31 as a pale yellow solid.

R*f* = 0.29 (EtOAc/pentane (40:60 (v/v)) (UV)).

HRMS-ESI(m/z) calc'd for C₁₂H₁₃IN₃O₄S⁺ [M+H]⁺, 421.9666; found, 421.9664; deviation: - 0.4 ppm.

### 1-(4-Iodophenyl)-1H-imidazole (32)

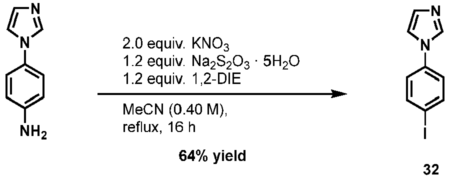

Under an ambient atmosphere, to a 4-mL borosilicate vial equipped with a magnetic stir bar was added 4-(1/7-imidazol-1-yl)aniline (79.6 mg, 0.500 mmol, 1.00 equiv.), KNO₃ (101 mg,1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 mL, c = 0.40 M). The vessel was sealed with a septum cap and heated at 80°C for 16 h in an aluminum block on a heating plate. Then, the reaction mixture was diluted with ethyl acetate (5 mL) and washed with a saturated aqueous Na₂S₂O₃ solution (7 mL). The aqueous phase was extracted with ethyl acetate (5 mL). The combined organic phase was dried over Na₂SO₄ and filtered. The resulting mixture was concentrated by rotary evaporation under reduced pressure. The residue was purified by silica gel chromatography by eluting with EtOAc to afford 86 mg (64%) of the title compound 32 as a colorless solid.

Rf= 0.20 (EtOAc/pentane (70:30 (v/v)) (UV)).

HRMS-ESI(m/z) calc'd for C₉H₈IN₂⁺ [M+H]⁺, 270.9727; found, 270.9725; deviation: -0.8 ppm.

### 2,5-dibromo-1,3-difluorobenzene (33)

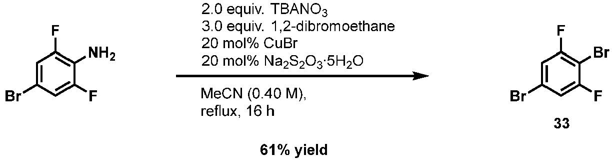

Under an ambient atmosphere, to a 4-mL borosilicate vial equipped with a magnetic stir bar was added 4-bromo-2,6-difluoroaniline (20.8 mg, 0.100 mmol, 1.00 equiv.), CuBr (2.8 mg, 0.020 mmol, 20 mol%), TBANO₃ (60.9 mg, 0.200 mmol, 2.00 equiv.), Na₂S₂O₃·5H₂O (5.0 mg, 0.020 mmol, 20 mol%) and acetonitrile (1.3 mL, c = 0.40 M). The vessel was sealed with a septum cap and heated at 80°C for 16 h in an aluminum block on a heating plate. After cooling to 25°C, the mixture was filtered through a 3 cm silica plug, eluted with 4 mL of CH₂Cl₂/pentane (1:1 (v/v)). The resulting mixture was concentrated by rotary evaporation under reduced pressure. The yield was determined by ¹H-NMR spectroscopy at 500 MHz and 298 K by dissolving the residue of the reaction mixture in 0.5 mL of CDCl₃, and using CH₂Br₂ as internal standard (7.0 µL, 17 mg, 0.10 mmol, 1.0 equiv.). The integration of the CH₂Br₂ signal at 4.96 ppm (s, 2H) was compared to the signal of 33 at 7.18 ppm (d, 2H).

**Note:** Due to the volatility of the product the compound was not isolated. The analytical data is consistent with data reported in the literature. Signal at 5.30 ppm corresponds to CH₂Cl₂, signals at 3.98 ppm, 3.57 ppm, 1.86 ppm, and 1.28 ppm correspond to TBA⁺; signal at 3.67 ppm corresponds to 1,2-DBE; signal at 2.05 ppm corresponds to MeCN.

HRMS-EI(m/z) calc'd for C₆H₂F₂Br₂⁺ [M]⁺, 269.8486; found, 269.8490; deviation: +1.3 ppm.

### Riluzole-derived hetaryl chloride 34

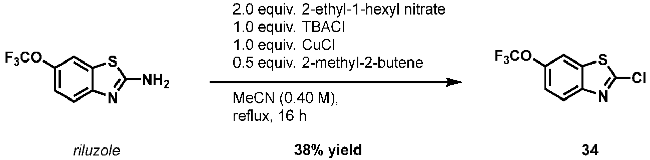

Under an ambient atmosphere, to a 4-mL borosilicate vial equipped with a magnetic stir bar was added riluzole (117 mg, 0.500 mmol, 1.00 equiv.), CuCI (49.5 mg, 0.500 mmol, 1.00 equiv.), TBACI (139 mg, 0.500 mmol, 1.00 equiv.), and acetonitrile (1.3 mL, c = 0.40 M). Then, 2-ethyl-1-hexylnitrate (188 µL, 181 mg, 1.00 mmol, 2.00 equiv.), and 2-methyl-2-butene (27 µL, 18 mg, 0.25 mmol, 0.50 equiv.) were added to the reaction mixture via a Hamilton syringe. The vessel was sealed with a septum cap and heated at 80°C for 16 h in an aluminum block on a heating plate. Then, the reaction mixture was allowed to cool to 23°C, diluted with ethyl acetate (5 mL), and washed twice with a saturated aqueous Na₂S₂O₃ solution (2 × 7 mL). The combined organic phase was dried over Na₂SO₄ and filtered. Then, the resulting filtrate was concentrated by rotary evaporation under reduced pressure. The residue was purified by silica gel chromatography by eluting with pentane to afford 48 mg (38%) of the title compound 34 as a colorless oil.

R*f* = 0.50 (EtOAc/pentane (5:95 (v/v)) (UV)).

HRMS-EI(m/z) calc'd for C₈H₃NOSF₃Cl⁺ [M]⁺, 252.9571; found, 252.9574; deviation: +1.5 ppm.

### Riluzole-derived hetaryl iodide 35

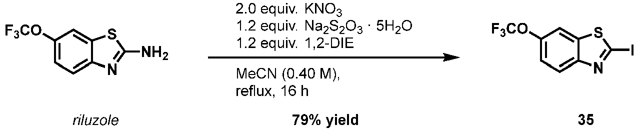

Under an ambient atmosphere, to a 4-mL borosilicate vial equipped with a magnetic stir bar was added riluzole (117 mg, 0.500 mmol, 1.00 equiv.), KNO₃ (101 mg, 1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 mL, c = 0.40 M). The vessel was sealed with a septum cap and heated at 80°C for 16 h in an aluminum block on a heating plate. Then, the reaction mixture was diluted with ethyl acetate (5 mL) and washed with a saturated aqueous Na₂S₂O₃ solution (7 mL). The aqueous phase was extracted with ethyl acetate (5 mL). The combined organic phase was dried over Na₂SO₄ and filtered. The resulting mixture was concentrated by rotary evaporation under reduced pressure. The residue was purified by silica gel chromatography by eluting with EtOAc/pentane (2:98 (v/v)) to afford 136 mg (79%) of the title compound 35 as a colorless solid.

Rf= 0.50 (EtOAc/pentane (5:95 (v/v)) (UV)).

HRMS-EI(m/z) calc'd for C₈H₃NOSF₃I⁺ [M]⁺, 344.3897; found, 344.8932; deviation: +1.6 ppm.

### Chloridazon-derived hetaryl chloride 36

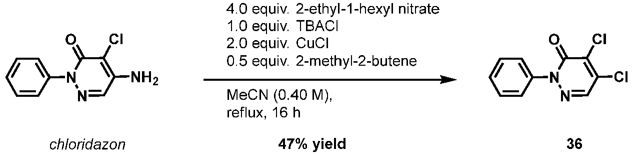

Under an ambient atmosphere, to a 4-mL borosilicate vial equipped with a magnetic stir bar was added chloridazon (111 mg, 0.500 mmol, 1.00 equiv.), CuCI (99 mg, 1.0 mmol, 2.0 equiv.), TBACI (139 mg, 0.500 mmol, 1.00 equiv.), and acetonitrile (1.3 mL, c = 0.40 M). Then, 2-ethyl-1-hexylnitrate (375 µL, 361 mg, 2.00 mmol, 4.00 equiv.), and 2-methyl-2-butene (27 µL, 18 mg, 0.25 mmol, 0.50 equiv.) were added to the reaction mixture via a Hamilton syringe. The vessel was sealed with a septum cap and heated at 80°C for 16 h in an aluminum block on a heating plate. Then, the reaction mixture was allowed to cool to 23°C, diluted with ethyl acetate (5 mL), and washed twice with a saturated aqueous Na₂S₂O₃ solution (2 × 7 mL). The combined organic phase was dried over Na₂SO₄ and filtered. Then, the resulting filtrate was concentrated by rotary evaporation under reduced pressure. The residue was purified by silica gel chromatography by eluting with EtOAc/pentane (10:90 (v/v)) to afford 56 mg (47%) of the title compound 36 as a colorless solid.

R*f* = 0.46 (EtOAc/pentane (20:80 (v/v)) (UV)).

HRMS-EI(m/z) calc'd for C₁₀H₆ON₂Cl₂⁺ [M]⁺, 239.9852; found, 239.9852; deviation: +0.0 ppm.

### Chloridazon-derived hetaryl iodide 37

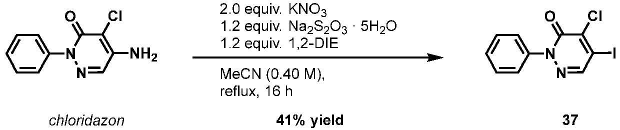

Under an ambient atmosphere, to a 4-mL borosilicate vial equipped with a magnetic stir bar was added chloridazon (111 mg, 0.500 mmol, 1.00 equiv.), KNO₃ (101 mg, 1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 mL, c = 0.40 M). The vessel was sealed with a septum cap and heated at 80°C for 16 h in an aluminum block on a heating plate. Then, the reaction mixture was diluted with ethyl acetate (5 mL) and washed with a saturated aqueous Na₂S₂O₃ solution (7 mL). The aqueous phase was extracted with ethyl acetate (5 mL). The combined organic phase was dried over Na₂SO₄ and filtered. The resulting mixture was concentrated by rotary evaporation under reduced pressure. The residue was purified by silica gel chromatography by eluting with EtOAc/pentane (10:90 (v/v)) to afford 68 mg (41%) of the title compound 37 as a colorless solid.

Rf= 0.54 (EtOAc/pentane (20:80 (v/v)) (UV)).

HRMS-EI(m/z) calc'd for C₁₀H₆N₂OClI⁺ [M]⁺, 331.9208; found, 331.9216; deviation: +2.5 ppm.

### Reduction of flutamide (S2)

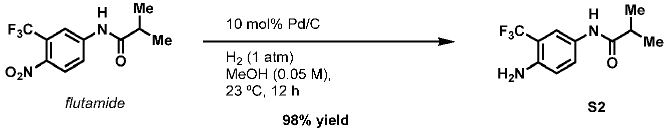

Under an Ar atmosphere, to a 250-mL round-bottomed Schlenk flask equipped with a magnetic stir bar was added flutamide (1.0 g, 3.6 mmol, 1.0 equiv.), and methanol (72 mL, c = 0.05 M). Then, the reaction mixture was stirred at 23°C for 5 min. Pd/C (385 mg, 0.362 mmol, 0.100 equiv.) was added under Ar atmosphere, and the flask was sealed with a septum. The atmosphere was evacuated and a balloon of H₂ was pierced through the septum to ensure a H₂ atmosphere. The reaction mixture was stirred on a hydrogen atmosphere. The reaction was monitored by TLC. After full consumption of the starting material (12 hours), the reaction mixture was filtered through celite. The resulting mixture was concentrated by rotary evaporation under reduced pressure. The residue was purified by silica gel chromatography by eluting with EtOAc/pentane (30:70 (v/v)) to afford 873 mg (98%) of the title compound S2 as a pink solid.

R*f* = 0.67 (EtOAc/pentane (60:40 (v/v)) (UV)).

HRMS-ESI(m/z) calc'd for NaC₁₁H₁₃N₂OF₃⁺ [M+Na]⁺, 269.0872; found, 269.0874; deviation: +0.7 ppm.

### Flutamide-derived aryl bromide 38

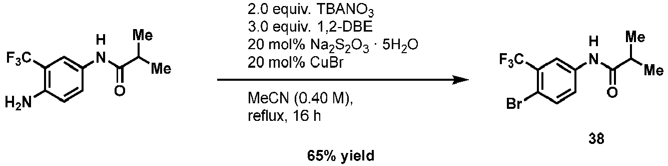

Under an ambient atmosphere, to a 4-mL borosilicate vial equipped with a magnetic stir bar was added S2 (123 mg, 0.500 mmol, 1.00 equiv), TBANO₃ (304 mg, 1.00 mmol, 2.00 equiv.), CuBr (13 mg, 0.10 mmol, 20 mol%), Na₂S₂O₃·5H₂O (34 mg, 0.10 mmol, 0.20 equiv.), and acetonitrile (1.3 mL, c = 0.40 M). Then, 1,2-dibromoethane (130µL, 282 mg, 1.50 mmol, 3.00 equiv.) was added to the reaction mixture via a Hamilton syringe. The vessel was sealed with a septum cap and heated at 80°C for 16 h in an aluminum block on a heating plate. Then, the reaction mixture was diluted with ethyl acetate (5 mL) and washed with a saturated aqueous Na₂S₂O₃ solution (7 mL). The aqueous phase was extracted with ethyl acetate (5 mL). The combined organic phase was dried over Na₂SO₄ and filtered. The resulting mixture was concentrated by rotary evaporation under reduced pressure. The residue was purified by silica gel chromatography by eluting with EtOAc/pentane (15:85 (v/v)) to afford 101 mg (65%) of the title compound 38 as a colorless solid.

R*f* = 0.59 (EtOAc/pentane (30:70 (v/v)) (UV)).

HRMS-EI(m/z) calc'd for C₁₁H₁₁NOF₃Br⁺ [M]⁺, 308.9971; found, 308.9975; deviation: +1.5 ppm.

### Flutamide-derived aryl iodide 39

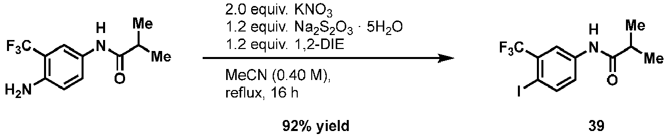

Under an ambient atmosphere, to a 4-mL borosilicate vial equipped with a magnetic stir bar was added S2 (123 mg, 0.500 mmol, 1.00 equiv), KNO₃ (101 mg, 1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 mL, c = 0.40 M). The vessel was sealed with a septum cap and heated at 80°C for 16 h in an aluminum block on a heating plate. Then, the reaction mixture was diluted with ethyl acetate (5 mL) and washed with a saturated aqueous Na₂S₂O₃ solution (7 mL). The aqueous phase was extracted with ethyl acetate (5 mL). The combined organic phase was dried over Na₂SO₄ and filtered. The resulting mixture was concentrated by rotary evaporation under reduced pressure. The residue was purified by silica gel chromatography by eluting with EtOAc/pentane (15:85 (v/v)) to afford 164 mg (92%) of the title compound 39 as a colorless solid.

Rf= 0.59 (EtOAc/pentane (30:70 (v/v)) (UV)).

HRMS-ESI(m/z) calc'd for C₁₁H₁₂F₃INO [M+H]⁺, 357.9910; found, 357.9910; deviation: +0.1 ppm.

### 4-Chloro-3,5-dimethylbenzonitrile (40)

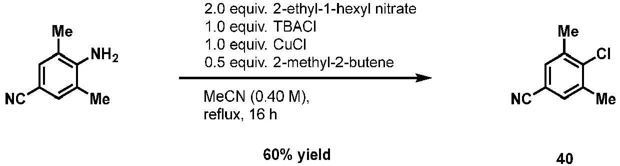

Under an ambient atmosphere, to a 4-mL borosilicate vial equipped with a magnetic stir bar was added 4-amino-3,5-dimethylbenzonitrile (73 mg, 0.50 mmol, 1.0 equiv.), CuCI (50 mg, 0.50 mmol, 1.0 equiv.), and acetonitrile (1.3 mL, c = 0.40 M). Then, 2-ethyl-1-hexylnitrate (188 µL, 181 mg, 1.00 mmol, 2.00 equiv.), and 2-methyl-2-butene (27 µL, 18 mg, 0.25 mmol, 0.50 equiv.) were added to the reaction mixture via a Hamilton syringe. The vessel was sealed with a septum cap and heated at 80°C for 16 h in an aluminum block on a heating plate. Then, the reaction mixture was allowed to cool to 23°C, diluted with ethyl acetate (5 mL), and washed twice with a saturated aqueous Na₂S₂O₃ solution (2 × 7 mL). The combined organic phase was dried over Na₂SO₄ and filtered. Then, the resulting filtrate was concentrated by rotary evaporation under reduced pressure. The residue was purified by silica gel chromatography by eluting with EtOAc/pentane (5:95 (v/v)) to afford 50 mg (60%) of the title compound 40 as a colorless solid.

R*f* = 0.43 (EtOAc/pentane (5:95 (v/v)) (UV)).

HRMS-EI(m/z) calc'd for C₉H₈NCl⁺ [M]⁺, 165.0340; found, 165.0343; deviation: +1.7 ppm.

### 4-Bromo-3,5-dimethylbenzonitrile (41)

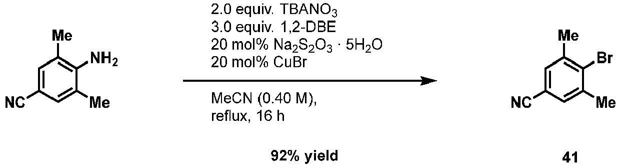

Under an ambient atmosphere, to a 4-mL borosilicate vial equipped with a magnetic stir bar was added 4-amino-3,5-dimethylbenzonitrile (73 mg, 0.50 mmol, 1.0 equiv.), TBANO₃ (304 mg, 1.00 mmol, 2.00 equiv.), CuBr (13 mg, 0.10 mmol, 20 mol%), Na₂S₂O₃·5H₂O (34 mg, 0.10 mmol, 0.20 equiv.), and acetonitrile (1.3 mL, c = 0.40 M). Then, 1,2-dibromoethane (130 µL, 282 mg, 1.50 mmol, 3.00 equiv.) was added to the reaction mixture via a Hamilton syringe. The vessel was sealed with a septum cap and heated at 80°C for 16 h in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with EtOAc/pentane (5:95 (v/v)) to afford 97 mg (92%) of the title compound 41 as a colorless solid.

Rf= 0.43 (EtOAc/pentane (5:95 (v/v)) (UV)).

HRMS-EI(m/z) calc'd for C₉H₈NBr⁺ [M]⁺, 208.9835; found, 208.9836; deviation: +0.6 ppm.

### 4-lodo-3,5-dimethylbenzonitrile (42)

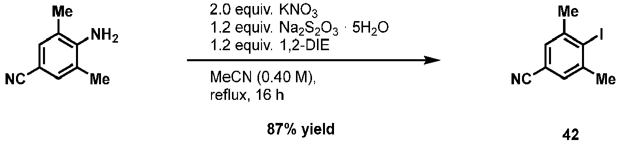

Under an ambient atmosphere, to a 4-mL borosilicate vial equipped with a magnetic stir bar was added 4-amino-3,5-dimethylbenzonitrile (73 mg, 0.50 mmol, 1.0 equiv.), KNO₃ (101 mg, 1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 mL, c = 0.40 M). The vessel was sealed with a septum cap and heated at 80°C for 16 h in an aluminum block on a heating plate. Then, the reaction mixture was diluted with ethyl acetate (5 mL) and washed with a saturated aqueous Na₂S₂O₃ solution (7 mL). The aqueous phase was extracted with ethyl acetate (5 mL). The combined organic phase was dried over Na₂SO₄ and filtered. The resulting mixture was concentrated by rotary evaporation under reduced pressure. The residue was purified by silica gel chromatography by eluting with pentane to afford 112 mg (87%) of the title compound 42 as a colorless solid.

R*f* = 0.43 (EtOAc/pentane (5:95 (v/v)) (UV)).

HRMS-EI(m/z) calc'd for C₉H₈NI⁺ [M]⁺, 256.9696; found, 256.9698; deviation: +1.0 ppm.

### 2,4-Dichlorobenzonitrile (43)

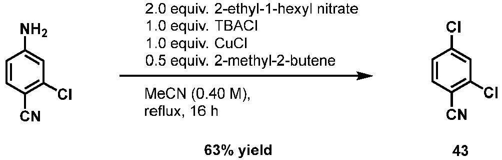

Under an ambient atmosphere, to a 4-mL borosilicate vial equipped with a magnetic stir bar was added 4-amino-2-chlorobenzonitrile (76 mg, 0.50 mmol, 1.0 equiv.), CuCI (50 mg, 0.50 mmol, 1.0 equiv.), TBACI (139 mg, 0.500 mmol, 1.00 equiv.), and acetonitrile (1.3 mL, c = 0.40 M). Then, 2-ethyl-1-hexylnitrate (188 µL, 181 mg, 1.00 mmol, 2.00 equiv.), and 2-methyl-2-butene (27 µL, 18 mg, 0.25 mmol, 0.50 equiv.) were added to the reaction mixture via a Hamilton syringe. The vessel was sealed with a septum cap and heated at 80°C for 16 h in an aluminum block on a heating plate. Then, the reaction mixture was allowed to cool to 23°C, diluted with ethyl acetate (5 mL), and washed twice with a saturated aqueous Na₂S₂O₃ solution (2 × 7 mL). The combined organic phase was dried over Na₂SO₄ and filtered. Then, the resulting filtrate was concentrated by rotary evaporation under reduced pressure. The residue was purified by silica gel chromatography by eluting with pentane to afford 54 mg (63%) of the title compound 43 as a colorless solid.

R*f* = 0.70 (EtOAc/pentane (5:95 (v/v)) (UV)).

HRMS-EI(m/z) calc'd for C₇H₃N₁Cl₂⁺ [M]⁺, 170.9639; found, 170.9637; deviation: -1.1 ppm.

### 4-Bromo-2-chlorobenzonitrile (44)

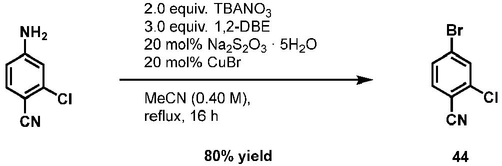

Under an ambient atmosphere, to a 4-mL borosilicate vial equipped with a magnetic stir bar was added 4-amino-2-chlorobenzonitrile (76 mg, 0.50 mmol, 1.0 equiv.), TBANO₃ (304 mg, 1.00 mmol, 2.00 equiv.), CuBr (13 mg, 0.10 mmol, 20 mol%), Na₂S₂O₃·5H₂O (34 mg, 0.10 mmol, 0.20 equiv.), and acetonitrile (1.3 mL, c = 0.40 M). Then, 1,2-dibromoethane (130 µL, 282 mg, 1.50 mmol, 3.00 equiv.) was added to the reaction mixture via a Hamilton syringe. The vessel was sealed with a septum cap and heated at 80°C for 16 h in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with EtOAc/pentane (2:98 (v/v)) to afford 87 mg (80%) of the title compound 44 as a colorless solid.

Rf= 0.92 (EtOAc/pentane (10:90 (v/v)) (UV)).

HRMS-EI(m/z) calc'd for C₇H₃N₁ClBr⁺ [M]⁺, 214.9132; found, 214.9135; deviation: +1.2 ppm.

### 2-Chloro-4-iodobenzonitrile (45)

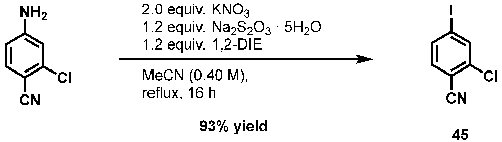

Under an ambient atmosphere, to a 4-mL borosilicate vial equipped with a magnetic stir bar was added 4-amino-3-chlorobenzonitrile (76 mg, 0.50 mmol, 1.0 equiv.), KNO₃ (101 mg, 1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 mL, c = 0.40 M). The vessel was sealed with a septum cap and heated at 80°C for 16 h in an aluminum block on a heating plate. Then, the reaction mixture was diluted with ethyl acetate (5 mL) and washed with a saturated aqueous Na₂S₂O₃ solution (7 mL). The aqueous phase was extracted with ethyl acetate (5 mL). The combined organic phase was dried over Na₂SO₄ and filtered. The resulting mixture was concentrated by rotary evaporation under reduced pressure. The residue was purified by silica gel chromatography by eluting with pentane to afford 123 mg (93%) of the title compound 45 as a colorless solid.

Rf= 0.50(EtOAc/pentane (10:90 (v/v)) (UV)).

HRMS-ESI(m/z) calc'd for C₇H₄NClI⁺ [M+H]⁺, 263.9072; found, 263.9072; deviation: +0.1 ppm.

### (4-Chlorophenyl)(phenyl)methanone (46)

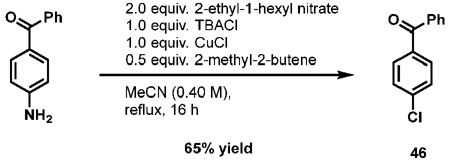

Under an ambient atmosphere, to a 4-mL borosilicate vial equipped with a magnetic stir bar was added (4-aminophenyl)(phenyl)methanone (99 mg, 0.50 mmol, 1.0 equiv), CuCI (50 mg, 0.50 mmol, 1.0 equiv.), TBACI (139 mg, 0.500 mmol, 1.00 equiv.), and acetonitrile (1.3 mL, *c* = 0.40 M). Then, 2-ethyl-1-hexylnitrate (188 µL, 181 mg, 1.00 mmol, 2.00 equiv.), and 2-methyl-2-butene (27 µL, 18 mg, 0.25 mmol, 0.50 equiv.) were added to the reaction mixture via a Hamilton syringe. The vessel was sealed with a septum cap and heated at 80°C for 16 h in an aluminum block on a heating plate. Then, the reaction mixture was allowed to cool to 23°C, diluted with ethyl acetate (5 mL), and washed twice with a saturated aqueous Na₂S₂O₃ solution (2 × 7 mL). The combined organic phase was dried over Na₂SO₄ and filtered. Then, the resulting filtrate was concentrated by rotary evaporation under reduced pressure. The residue was purified by silica gel chromatography by eluting with EtOAc/pentane (5:95 (v/v)) to afford 70 mg (65%) of the title compound 36 as a colorless solid.

R*f* = 0.36 (EtOAc/pentane (5:95 (v/v)) (UV)).

HRMS-EI(m/z) calc'd for C₁₃H₉OCl⁺ [M]⁺, 216.0336; found, 216.0339; deviation: +1.2 ppm.

### (4-Bromophenyl)(phenyl)methanone (47)

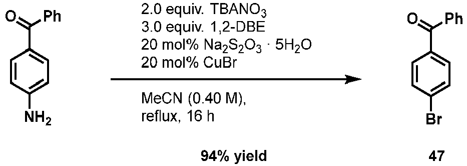

Under an ambient atmosphere, to a 4-mL borosilicate vial equipped with a magnetic stir bar was added (4-aminophenyl)(phenyl)methanone (99 mg, 0.50 mmol, 1.0 equiv), TBANO₃ (304 mg,1.00 mmol, 2.00 equiv.), CuBr (13 mg, 0.10 mmol, 20 mol%), Na₂S₂O₃·5H₂O (34 mg, 0.10 mmol, 0.20 equiv.), and acetonitrile (1.3 mL, c = 0.40 M). Then, 1,2-dibromoethane (130 µL, 282 mg, 1.50 mmol, 3.00 equiv.) was added to the reaction mixture via a Hamilton syringe. The vessel was sealed with a septum cap and heated at 80°C for 16 h in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with EtOAc/pentane (5:95 (v/v)) to afford 123 mg (94%) of the title compound 47 as a colorless solid.

R*f* = 0.36 (EtOAc/pentane (5:95 (v/v)) (UV)).

HRMS-EI(m/z) calc'd for C₁₃H₉OBr⁺ [M]⁺, 259.9831; found, 259.9834; deviation: +1.0 ppm.

### 3-Chloro-2-methoxydibenzofuran (48)

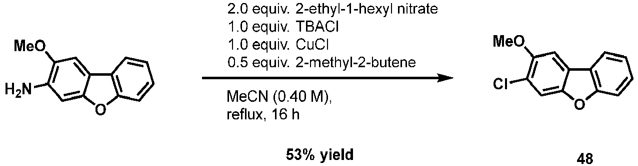

Under an ambient atmosphere, to a 4-mL borosilicate vial equipped with a magnetic stir bar was added 3-amino-2-methoxydibenzofuran (107 mg, 0.500 mmol, 1.00 equiv.), CuCI (50 mg, 0.50 mmol, 1.0 equiv.), and acetonitrile (1.3 mL, c = 0.40 M). Then, 2-ethyl-1-hexylnitrate (188 µL, 181 mg, 1.00 mmol, 2.00 equiv.), and 2-methyl-2-butene (27 µL, 18 mg, 0.25 mmol, 0.50 equiv.) were added to the reaction mixture via a Hamilton syringe.

The vessel was sealed with a septum cap and heated at 80°C for 16 h in an aluminum block on a heating plate. Then, the reaction mixture was allowed to cool to 23°C, diluted with ethyl acetate (5 mL), and washed twice with a saturated aqueous Na₂S₂O₃ solution (2 × 7 mL). The combined organic phase was dried over Na₂SO₄ and filtered. Then, the resulting filtrate was concentrated by rotary evaporation under reduced pressure. The residue was purified by silica gel chromatography by eluting with EtOAc/pentane (5:95 (v/v)) to afford 61 mg (53%) of the title compound 48 as a colorless solid.

Rf= 0.72 (EtOAc/hexanes (10:90 (v/v)) (UV)).

HRMS-EI(m/z) calc'd for C₁₃H₉O₂Cl⁺ [M]⁺, 232.0285; found, 232.0290; deviation: +1.8 ppm.

### 3-Bromo-2-methoxydibenzofuran (49)

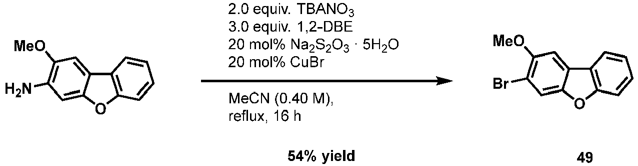

Under an ambient atmosphere, to a 4-mL borosilicate vial equipped with a magnetic stir bar was added 3-amino-2-methoxydibenzofuran (107 mg, 0.500 mmol, 1.00 equiv.), TBANO₃ (304 mg, 1.00 mmol, 2.00 equiv.), CuBr (13 mg, 0.10 mmol, 20 mol%), Na₂S₂O₃·5H₂O (34 mg, 0.10 mmol, 0.20 equiv.), and acetonitrile (1.3 mL, c = 0.40 M). Then, 1,2-dibromoethane (130 µL, 282 mg, 1.50 mmol, 3.00 equiv.) was added to the reaction mixture via a Hamilton syringe. The vessel was sealed with a septum cap and heated at 80°C for 16 h in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with EtOAc/pentane (5:95 (v/v)) to afford 75 mg (54%) of the title compound 49 as a colorless solid.

Rf= 0.68 (EtOAc/hexanes (10:90 (v/v)) (UV)).

HRMS-EI(m/z) calc'd for C₁₃H₉O₂Br⁺ [M]⁺, 275.9781; found, 275.9785; deviation: +1.7 ppm.

### 3-lodo-2-methoxydibenzofuran (50)

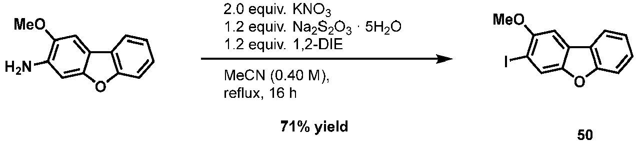

Under an ambient atmosphere, to a 4-mL borosilicate vial equipped with a magnetic stir bar was added 3-amino-2-methoxydibenzofuran (107 mg, 0.500 mmol, 1.00 equiv.), KNO₃ (101 mg, 1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 mL, c = 0.40 M). The vessel was sealed with a septum cap and heated at 80°C for 16 h in an aluminum block on a heating plate. Then, the reaction mixture was diluted with ethyl acetate (5 mL) and washed with a saturated aqueous Na₂S₂O₃ solution (7 mL). The aqueous phase was extracted with ethyl acetate (5 mL). The combined organic phase was dried over Na₂SO₄ and filtered. The resulting mixture was concentrated by rotary evaporation under reduced pressure. The residue was purified by silica gel chromatography by eluting with pentane to afford 115 mg (71%) of the title compound 50 as a colorless solid.

Rf= 0.68 (EtOAc/hexanes (10:90 (v/v)) (UV)).

HRMS-EI(m/z) calc'd for C₁₃H₉O₂I⁺ [M]⁺, 323.9642; found, 323.9647; deviation: +1.5 ppm.

### Deaminative halogenation of anilines whose diazonium salts are explosive

**Caution:** The diazonium salts derived from the anilines functionalized in this section have been reported to decompose violently or to detonate (as stated in Fig. 4B). Using our conditions, the diazonium salt is only formed as a short-lived intermediate and the risk is diminished. We have never experienced any explosion or violent decomposition, but a blast shield must be used to minimize personal damage in case of an accident.

### 2-lodobenoic acid (52)

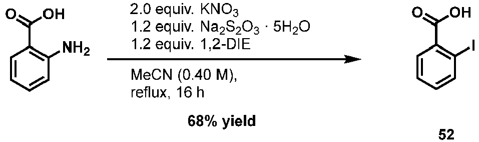

Under an ambient atmosphere, to a 4-mL borosilicate vial equipped with a magnetic stir bar was added anthranilic acid (69 mg, 0.50 mmol, 1.0 equiv.), KNO₃ (101 mg, 1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 mL, c = 0.40 M). The vessel was sealed with a septum cap and heated at 80°C for 16 h in an aluminum block on a heating plate. Then, the reaction mixture was allowed to cool to 23°C, diluted with ethyl acetate (20 mL), and washed with a saturated aqueous Na₂S₂O₃ solution (7 mL). The aqueous phase was extracted with ethyl acetate (20 mL). The combined organic phase was dried over Na₂SO₄ and filtered. The resulting mixture was concentrated by rotary evaporation under reduced pressure. The residue was purified by silica gel chromatography by eluting with AcOH/EtOAc/pentane (1:49:50 (v/v)) to afford 84 mg (68%) of the title compound 52 as a colorless solid.

Rf= 0.46 (AcOH/EtOAc/pentane = 1:49:50, v/v (UV)).

HRMS-EI(m/z) calc'd for C₇H₅IO₂⁺ [M]⁺, 247.9329; found, 247.9332; deviation: +1.1 ppm.

### 1-lodo-3-methylbenzene (54)

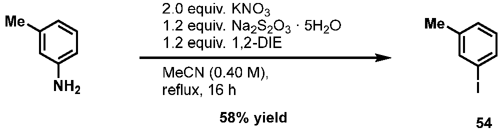

Under an ambient atmosphere, to a 4-mL borosilicate vial equipped with a magnetic stir bar was added KNO₃ (101 mg, 1.00 mmol, 2.00 equiv.), Na₂S₂O₃·5H₂O (149 mg, 0.600 mmol, 1.20 equiv.), 1,2-diiodoethane (169 mg, 0.600 mmol, 1.20 equiv), and acetonitrile (1.3 mL, c = 0.40 M). Then, meta-toluidine (55 µL, 0.500 mmol, 1.00 equiv.) was added to the reaction mixture via a Hamilton syringe. The vessel was sealed with a septum cap and heated at 80°C for 16 h in an aluminum block on a heating plate. Then, the reaction mixture was allowed to cool to 23°C, diluted with dichloromethane (5 mL), and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with EtOAc/pentane (1:99 (v/v)) to afford 114 mg (91%) of the title compound 54 as a pale orange solid.

R*f* = 0.40 (in pentane (UV)).

HRMS-EI(m/z) calc'd for C₇H₇I⁺ [M]⁺, 217.9587; found, 217.9587; deviation: +0.1 ppm.

### 3-Bromo-2-chloropyridine (56)

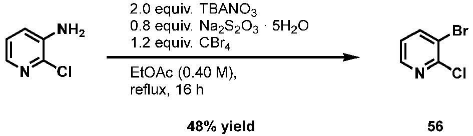

Under an ambient atmosphere, to a 4-mL borosilicate vial equipped with a magnetic stir bar was added 3-amino-2-chloropyridine (64 mg, 0.50 mmol, 1.0 equiv.), TBANO₃ (304 mg, 1.00 mmol, 2.00 equiv.), Na₂S₂O₃·5H₂O (99 mg, 0.60 mmol, 1.2 equiv.), tetrabromomethane (199 mg, 0.600 mmol, 1.20 equiv), and ethylacetate (1.3 mL, c = 0.40 M). The vessel was sealed with a septum cap and heated at 80°C for 40 minutes in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with EtOAc/pentane (5:95 (v/v)) to afford 34 mg (48%) of the title compound 56 as a colorless oil.

R*f* = 0.45 (EtOAc/pentane (10:90 (v/v)) (UV)).

HRMS-EI(m/z) calc'd for C₅H₃NClBr⁺ [M]⁺, 190.9132; found, 190.9135; deviation: +1.6 ppm.

### 4-Chloro-4-nitrobenzene (58)

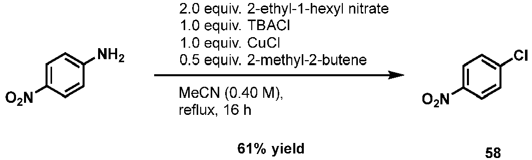

Under an ambient atmosphere, to a 4-mL borosilicate vial equipped with a magnetic stir bar was added 4-nitroaniline (69 mg, 0.50 mmol, 1.0 equiv.), CuCI (50 mg, 0.50 mmol, 1.0 equiv.), TBACI (139 mg, 0.500 mmol, 1.00 equiv.), and acetonitrile (1.3 mL, c = 0.40 M). Then, 2-ethyl-1-hexylnitrate (188 µL, 181 mg, 1.00 mmol, 2.00 equiv.), and 2-methyl-2-butene (27 µL, 18 mg, 0.25 mmol, 0.50 equiv.) were added to the reaction mixture via a Hamilton syringe. The vessel was sealed with a septum cap and heated at 80°C for 16 h in an aluminum block on a heating plate. Then, the reaction mixture was allowed to cool to 23°C, diluted with ethyl acetate (5 mL), and washed twice with a saturated aqueous Na₂S₂O₃ solution (2 × 7 mL). The combined organic phase was dried over Na₂SO₄ and filtered. The resulting mixture was concentrated by rotary evaporation under reduced pressure. The residue was purified by silica gel chromatography by eluting with EtOAc/pentane (5:95 (v/v)) to afford 48 mg (61%) of the title compound 58 as a colorless solid.

R*f* = 0.21 (EtOAc/pentane (2:98 (v/v)) (UV)).

HRMS-EI(m/z) calc'd for C₆H₄NO₂Cl⁺ [M]⁺, 156.9925; found, 156.9927; deviation: + 1.2 ppm.

### 1-(Azidomethyl)-2-chlorobenzene (60)

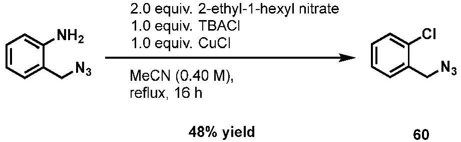

Under an ambient atmosphere, to a 4-mL borosilicate vial equipped with a magnetic stir bar was added 2-(azidomethyl)aniline (74 mg, 0.50 mmol, 1.0 equiv), CuCI (50 mg, 0.50 mmol, 1.0 equiv.), TBACI (139 mg, 0.500 mmol, 1.00 equiv.), and acetonitrile (1.3 mL, c = 0.40 M). Then, 2-ethyl-1-hexylnitrate (188 µL, 181 mg, 1.00 mmol, 2.00 equiv.), was added to the reaction mixture via a Hamilton syringe. The vessel was sealed with a septum cap and heated at 80°C for 16 h in an aluminum block on a heating plate. Then, the reaction mixture was allowed to cool to 23°C, diluted with ethyl acetate (5 mL), and washed twice with a saturated aqueous Na₂S₂O₃ solution (2 × 7 mL). The combined organic phase was dried over Na₂SO₄ and filtered. The resulting mixture was concentrated by rotary evaporation under reduced pressure. The residue was purified by silica gel chromatography by eluting with pentane to afford 41 mg (48%) of the title compound 60 as a colorless oil.

R*f* = 0.50 (in pentane (UV)).

HRMS-EI(m/z) calc'd for C₇H₆N₃Cl⁺ [M]⁺, 167.0245; found, 167.0245; deviation: +0.6 ppm.

### 2-Bromo-1-chloro-3,5-dinitrobenzene (62)

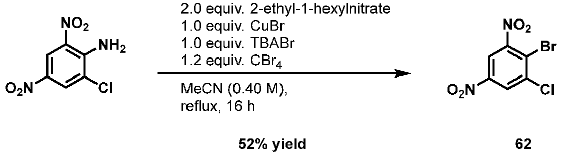

Under an ambient atmosphere, to a 4-mL borosilicate vial equipped with a magnetic stir bar was added 6-chloro-2,4-dinitroaniline (109 mg, 0.500 mmol, 1.00 equiv.), TBANO₃ (304 mg, 1.00 mmol, 2.00 equiv.), Na₂S₂O₃·5H₂O (99 mg, 0.60 mmol, 1.2 equiv.), tetrabromomethane (199 mg, 0.600 mmol, 1.20 equiv), and ethylacetate (1.3 mL, c = 0.40 M). The reaction mixture was stirred in a sealed vial at 80°C for 16 h. The vessel was sealed with a septum cap and heated at 80°C for 16 h in an aluminum block on a heating plate. Then, the reaction mixture was diluted with dichloromethane (5 mL) and washed with a saturated aqueous Na₂S₂O₃ solution (7 mL). The aqueous phase was extracted with dichloromethane (5 mL). The combined organic phase was dried over Na₂SO₄ and filtered. The resulting mixture was concentrated by rotary evaporation under reduced pressure. The residue was purified by silica gel chromatography by eluting with EtOAc/pentane (5:95 (v/v)) to afford 73 mg (52%) of the title compound 62 as a colorless solid.

R*_{f}* = 0.54 (EtOAc/pentane (10:90 (v/v)) (UV)).

HRMS-EI(m/z) calc'd for C₆H₂N₂O₄ClBr⁺ [M]⁺, 279.8881; found, 279.8886; deviation: +1.8 ppm.

### Summary

As detailed above, the present invention provides that nitrate reduction of inexpensive and readily available nitrate salts and nitrate esters allows the direct functionalization such as a deaminative of anilines and aminoheterocycles without the need for modern and specialized reagents. The fundamentally different mechanism of nitrate reduction over nitrite protonation allows diazonium chemistry at elevated temperatures, and the functionalization of substrates bearing energetic or sensitive functional groups. The immediate consequence as detailed in the present application here is a safer and higher yielding halogenation, yet, the new mechanism manifold may also provide a more general platform for the development of diazonium chemistry beyond current reach due to the lack of required high energy intermediate accumulation as was the case since the nineteenth century.

## Claims

1. Process for the functionalization of an aromatic or heteroaromatic amino compound wherein an aromatic or heteroaromatic amino compound is reacted with a compound pair, selected from a pair of an (alkali metal or ammonium) nitrate or mixtures thereof and a sulfur oxygen compound as reductant or mixtures thereof or a pair of a nitrate ester of an alkyl alcohol and a dihalocuprate, in the presence of a halogen source, selected from halogen salts or halogen alkyl compounds, whereby a halogenated aromatic or heteroaromatic compound is obtained.

2. Process for the functionalization of an aromatic or heteroaromatic amino compound according to claim 1, wherein an aromatic or heteroaromatic amino compound is reacted with an (alkali metal or ammonium) nitrate or mixtures thereof and a sulfur oxygen compound as reductant or mixtures thereof in the presence of a halogen source, selected from halogen salts or halogen alkyl compounds, whereby a halogenated aromatic or heteroaromatic compound is obtained.

3. Process for the functionalization of an aromatic or heteroaromatic amino compound according to claim 1 or 2, wherein the sulfur-oxygen reductant is selected from alkali or ammonium salts of thiosulfuric acid, thiosulfates, polythionates, dithionates, trithionates, tetrathionates, sulfites, bisulfites, sulfur dioxide, or metabisulfites, or mixtures thereof.

4. Process for the functionalization of an aromatic or heteroaromatic amino compound according to claim 1, wherein an aromatic or heteroaromatic amino compound is reacted with a nitrate ester of an alkyl alcohol and a dihalocuprate, in the presence of a halogen source, selected from halogen salts or halogen alkyl compounds, whereby a halogenated aromatic or heteroaromatic compound is obtained.

5. Process for the functionalization of an aromatic or heteroaromatic amino compound according to claim 1 or 4, wherein the nitrate ester of the alkyl alcohol is selected from nitrates of C4 bis C10 alkyl alcohols, and is preferably 2-ethyl hexyl nitrate.

6. Process for the functionalization of an aromatic or heteroaromatic amino compound according to any one of the preceding claims, wherein the reaction is carried out in an optionally aqueous polar aprotic solvent such as, but not limited to: acetonitrile, butyronitrile, ethylacetate, chloroform, 1 ,2-dichloroethane, dichloromethane, acetone, or mixtures thereof.

7. Process for the functionalization of an aromatic or heteroaromatic amino compound according to any one of the preceding claims, wherein the process is carried out in a temperature range from 40°C to 120°C, preferably at the reflux temperature of the used solvent.

8. Process for the functionalization of an aromatic or heteroaromatic amino compound according to any one of the preceding claims, wherein the aromatic or heteroaromatiic amino compound is selected from C5 to C20 aryl or heteroaryl compounds.

9. Process for the functionalization of an aromatic or heteroaromatic amino compound according to any one of the preceding claims, wherein the functionalization comprises a deaminative halogenation aromatic or heteroaromatiic amino compound.
